# EUROPEAN PATENT APPLICATION

(11) **EP 1 510 583 A1**
(43) Date of publication of application: **02.03.2005**
(21) Application number: 03728047.6
(22) Date of filing: 08.05.2003
(51) Int. Cl.: C12N 15/54, C12N 15/60, C12N 9/12, C12N 9/88, C12N 1/21, C12P 7/42

(54) **PROCESS FOR PRODUCING MEVALONIC ACID**

(30) Priority: 10.05.2002 JP 2002135073
(71) Applicant: KYOWA HAKKO KOGYO CO., LTD., Chiyoda-ku, Tokyo 100-8185 (JP)
(72) Inventor: Tabata, Kazuhiko, Technical Research Laboratories, Hofu-shi, Yamaguchi 747-8522 (JP); Hashimoto, Shin-ichi, Technical Res. Laboratories, Hofu-shi, Yamaguchi 747-8522 (JP)
(74) Representative: VOSSIUS & PARTNER
(86) International application number: PCT/JP2003/005765
(87) International publication number: WO 2003/095651

(57) **Abstract**

A microorganism having the ability to biosynthesize mevalonic acid from acetyl-CoA is obtained by transfecting a DNA participating in biosynthesis of mevalonic acid from acetyl-CoA into a host microorganism, preferably into a microorganism having only a non-mevalonic acid pathway, to express the DNA therein. Mevalonic acid can be efficiently produced by culturing the microorganism, and recovering mevalonic acid that is produced and accumulated in a large amount in the culture.

## Description

### Technical Field

The present invention relates to a recombinant microorganism which is provided an ability to produce mevalonic acid, and a process for producing mevalonic acid using the microorganism.

### Background Art

Mevalonic acid is a substance isolated first by Wright et al., [J. Am. Chem. Soc., 78, 5273, 1956]. In an organism, mevalonic acid has been known to be an intermediate in biosynthesis of isopentenyl pyrophosphate, which provides a basis of the structure of various types of isoprenoid compounds that include cholesterol [Harper's review of Biochemistry (19th edition), Lange Medical Publications (1983)]. Isopentenyl pyrophosphate biosynthetic pathway via mevalonic acid is referred to as a mevalonic acid pathway, which is as follows. Acetyl coenzyme A (hereinafter, abbreviated as acetyl-CoA), which is generated by glycolysis and the like, is converted to acetoacetyl-CoA by the activity of acetyl-CoA acetyltransferase, and further converted to 3-hydroxy-3-methylglutaryl coenzyme A (hereinafter, abbreviated as HMG-CoA) by the activity of HMG-CoA synthase, followed by production of mevalonic acid by the activity of HMG-CoA reductase. Mevalonic acid is converted to mevalonate-5-phosphate by the activity of mevalonate kinase, and then converted to mevalonate-5-diphosphate by the activity of phosphomevalonate kinase. Finally, isopentenyl pyrophosphate is produced by the activity of diphosphomevalonate decarboxylase.

Although mevalonic acid exhibits two types of structures, an acid form and a lactone form, it has been known that they can be converted each other.

Mevalonic acid is used as a growth promoter for a variety of microorganisms, plants and the like, and is used as synthetic precursors of pyrethroid pesticides, ubiquinone (respiratory coenzyme), dolichol (essential factor for biosynthesis of polysaccharides), lipid-soluble vitamin and the like. Furthermore, effects of mevalonic acid on the skin, such as the ability to impart adequate moisturized feel and softness (Japanese Published Unexamined Patent Application No. 099707/1992) and an anti-aging action (Japanese Published Unexamined Patent Application No. 338626/1998), were reported in recent years, and it is used as an ingredient of cosmetics. Conventionally, a chemically synthesized racemic form has been used in these researches, and it has been difficult to obtain naturally occurring mevalonic acid [R(+) form that is present in an organism].

Although production of naturally occurring mevalonic acid by a fermentation method was reported as an example in which Saccharomycopsis fibuligera NRRL Y-7069 is used [US 3617447; Appl. Microbiol., 16, 965 (1968)], the yield thereby is low, which may be just 700 to 1000 µg/mL, not leading to industrial production. Further, productivity is improved to approximately 20 g/L using Saccharomycopsis fibuligera IF00107 [J. Ferm. Bioeng., 86, 58 (1989)], ADK8107, ADK8108 (Japanese Published Unexamined Patent Application No. 210174/1991) that belong to the same species, more recently. However a cultivation time is as long as 200 hrs, and the yield from the sugar is low. Accordingly, it is far from an industrially useful process for the production.

### Disclosure of the Invention

A efficient process for producing mevalonic acid that can be practiced in industrial production has been desired.

In biosynthesis of isopentenyl pyrophosphate, a biosynthetic pathway not via mevalonic acid (non-mevalonic acid pathway) has been found in microorganisms and plants [Protein Nucleic acid and Enzyme, 42 , 2590 (1997)], in addition to a biosynthetic pathway via mevalonic acid (mevalonic acid pathway). The present inventors elaborately carried out the investigation on the basis of a hypothesis that a microorganism having only a non-mevalonic acid pathway but not having a mevalonic acid pathway does not metabolize mevalonic acid, thereby enabling extremely efficient production of mevalonic acid, and thus, the present invention was accomplished.

The present invention provides the following items (1) to (32).
(1) A process for producing mevalonic acid, which comprises:
   culturing a microorganism having the ability to biosynthesize mevalonic acid from acetyl coenzyme A (acetyl-CoA) in a medium, the microorganism being obtainable by transfecting a DNA participating in biosynthesis of mevalonic acid from acetyl-CoA into a host microorganism;
   allowing production and accumulation of mevalonic acid in the culture; and
   recovering mevalonic acid from the culture.
(2) The process according to the above item (1), wherein the host microorganism is a microorganism not having the ability to biosynthesize mevalonic acid from acetyl-CoA.
(3) The process according to the above item (2), wherein the microorganism not having the ability to biosynthesize mevalonic acid from acetyl-CoA is a microorganism not having a mevalonic acid pathway.
(4) The process according to any one of the above items (1) to (3), wherein the DNA to be transfected is one or more DNA(s) selected from the group consisting of a DNA encoding an enzyme having the activity to produce acetoacetyl-CoA from acetyl-CoA, a DNA encoding an enzyme having the activity to produce 3-hydroxy-3-methylglutaryl coenzyme A (HMG-CoA) from acetoacetyl-CoA, and a DNA encoding an enzyme having the activity to produce mevalonic acid from HMG-CoA.
(5) The process according to the above item (4), wherein the DNA encoding an enzyme having the activity to produce acetoacetyl-CoA from acetyl-CoA is acetyl-CoA acetyltransferase.
(6) The process according to the above item (4), wherein the DNA encoding an enzyme having the activity to produce HMG-CoA from acetoacetyl-CoA is HMG-CoA synthase.
(7) The process according to the above item (4), wherein the DNA encoding an enzyme having the activity to produce mevalonic acid from HMG-CoA is HMG-CoA reductase.
(8) The process according to the above item (4), wherein the DNA encoding an enzyme having the activity to produce acetoacetyl-CoA from acetyl-CoA and the DNA encoding an enzyme having the activity to produce mevalonic acid from HMG-CoA are a DNA encoding acetyl-CoA acetyltransferase/HMG-CoA reductase.
(9) The process according to any one of the above items (1) to (3), wherein the DNA to be transfected is a DNA encoding acetyl-CoA acetyltransferase, a DNA encoding HMG-CoA synthase and a DNA encoding HMG-CoA reductase.
(10) The process according to any one of the above items (1) to (3), wherein the DNA to be transfected is a DNA encoding acetyl-CoA acetyltransferase/HMG-CoA reductase and HMG-CoA synthase.
(11) The process according to any one of the above items (1) to (10), wherein the DNA is derived from one or more microorganism(s) selected from the group consisting of bacteria, mycobacteria, archaebacteria and yeast.
(12) The process according to any one of the above items (1) to (10), wherein the DNA is derived from a microorganism belonging to a genus selected from the group consisting of genus Enterococcus, genus Streptococcus, genus Staphylococcus, genus Lactococcus, genus Streptomyces, genus Methanothermobacter, genus Methanococcus, genus Pyrococcus, genus Thermoplasma and genus Saccharomyces.
(13) The process according to any one of the above items (1) to (3), wherein the DNA to be transfected is one or more DNA(s) selected from the group consisting of the following (a) to (j):
   (a) mvaE of Enterococcus faecalis;
   (b) mvaS of Enterococcus faecalis;
   (c) thiL of Lactococcus lactis;
   (d) mvaA of Lactococcus lactis;
   (e) hmcC of Lactococcus lactis;
   (f) a DNA encoding an amino acid sequence of SEQ ID NO: 2;
   (g) a DNA encoding an amino acid sequence of SEQ ID NO: 4;
   (h) a DNA encoding an amino acid sequence of SEQ ID NO: 12;
   (i) a DNA encoding an amino acid sequence of SEQ ID NO: 14; and
   (j) a DNA encoding an amino acid sequence of SEQ ID NO: 16.
(14) The process according to the above item (3), wherein the microorganism not having a mevalonic acid pathway is a microorganism belonging to a genus selected from the group consisting of genus Escherichia, genus Serratia, genus Bacillus, genus Brevibacterium, genus Corynebacterium, genus Microbacterium, genus Pseudomonas and genus Synechocystis.
(15). The process according to the above item (14), wherein the microorganism belonging to genus Escherichia is Escherichia coli.
(16) The process according to any one of the above items (1) to (3), wherein the microorganism having the ability to biosynthesize mevalonic acid from acetyl-CoA is a microorganism obtainable by transfecting a DNA participating in biosynthesis of mevalonic acid from acetyl-CoA into a host microorganism using an expression vector.
(17) A microorganism having the ability to biosynthesize mevalonic acid from acetyl-CoA and being obtainable by transfecting a DNA participating in biosynthesis of mevalonic acid from acetyl-CoA into a host microorganism.
(18) The microorganism according to the above item (17), wherein the host microorganism is a microorganism not having the ability to biosynthesize mevalonic acid from acetyl-CoA.
(19) The microorganism according to the above item (18), wherein the microorganism not having the ability to biosynthesize mevalonic acid from acetyl-CoA is a microorganism not having a mevalonic acid pathway.
(20) The microorganism according to any one of the above items (17) to (19), wherein the DNA to be transfected is one or more DNA(s) selected from the group consisting of a DNA encoding an enzyme having the activity to produce acetoacetyl-CoA from acetyl-CoA, a DNA encoding an enzyme having the activity to produce HMG-CoA from acetoacetyl-CoA, and a DNA encoding an enzyme having the activity to produce mevalonic acid from HMG-CoA.
(21) The microorganism according to the above item (20), wherein the DNA encoding an enzyme having the activity to produce acetoacetyl-CoA from acetyl-CoA is acetyl-CoA acetyltransferase.
(22) The microorganism according to the above item (20), wherein the DNA encoding an enzyme having the activity to produce HMG-CoA from acetoacetyl-CoA is HMG-CoA synthase.
(23) The microorganism according to the above item (20), wherein the DNA encoding an enzyme having the activity to produce mevalonic acid from HMG-CoA is HMG-CoA reductase.
(24) The microorganism according to the above item (20), wherein the DNA encoding an enzyme having the activity to produce acetoacetyl-CoA from acetyl-CoA and the DNA encoding an enzyme having the activity to produce mevalonic acid from HMG-CoA are a DNA encoding acetyl-CoA acetyltransferase/HMG-CoA reductase.
(25) The microorganism according to any one of the above items 17 to 19, wherein the DNA to be transfected is a DNA encoding acetyl-CoA acetyltransferase, a DNA encoding HMG-CoA synthase and a DNA encoding HMG-CoA reductase.
(26) The microorganism according to any one of the above items (17) to (19), wherein the DNA to be transfected is a DNA encoding acetyl-CoA acetyltransferase/HMG-CoA reductase and HMG-CoA synthase.
( 2 7 ) The microorganism according to any one of the above items (17) to (26), wherein the DNA is derived from one or more microorganism(s) selected from the group consisting of bacteria, mycobacteria, archaebacteria and yeast.
(28) The microorganism according to any one of the above items (17) to (26), wherein the DNA is derived from a microorganism belonging to a genus selected from the group consisting of genus Enterococcus, genus Streptococcus, genus Staphylococcus, genus Lactococcus, genus Lactobacillus, genus Streptomyces, genus Methanothermobacter, genus Methanococcus, genus Pyrococcus, genus Thermoplasma and genus Saccharomyces.
(29) The microorganism according to any one of the above items (17) to (19), wherein the DNA to be transfected is one or more DNA(s) selected from the group consisting of the following (a) to (j):
   (a) mvaE of Enterococcus faecalis;
   (b) mvaS of Enterococcus faecalis;
   (c) thiL of Lactococcus lactis;
   (d) mvaA of Lactococcus lactis;
   (e) hmcC of Lactococcus lactis;
   (f) a DNA encoding an amino acid sequence of SEQ ID NO: 2;
   (g) a DNA encoding an amino acid sequence of SEQ ID NO: 4;
   (h) a DNA encoding an amino acid sequence of SEQ ID NO: 12;
   (i) a DNA encoding an amino acid sequence of SEQ ID NO: 14; and
   (j) a DNA encoding an amino acid sequence of SEQ ID NO: 16.
(30) The microorganism according to the above item (19), wherein the microorganism not having a mevalonic acid pathway is a microorganism belonging to a genus selected from the group consisting of genus Escherichia, genus Serratia, genus Bacillus, genus Brevibacterium, genus Corynebacterium, genus Microbacterium, genus Pseudomonas and genus Synechocystis.
(31) The microorganism according to the above item (30), wherein the microorganism belonging to genus Escherichia is Escherichia coli.
(32) The microorganism according to any one of the above items (17) to (19), wherein the microorganism having the ability to biosynthesize mevalonic acid from acetyl-CoA is a microorganism obtainable by transfecting a DNA participating in biosynthesis of mevalonic acid from acetyl-CoA into a host microorganism using an expression vector.

The present invention is explained below in detail.

The microorganism not having the ability to biosynthesize mevalonic acid from acetyl-CoA herein means a microorganism lacking the ability due to having a mutation on a mevalonic acid pathway or a microorganism having only a non-mevalonic acid pathway.

The mevalonic acid herein may have either one of the two types of structures, i.e., the acid form and the lactone form.

Among the microorganisms lacking the ability due to having a mutation on the mevalonic acid pathway, preferred are microorganisms having a weak activity to metabolize mevalonic acid, or not having the activity.

Examples of the microorganism having only a non-mevalonic acid pathway include bacteria belonging to genus Escherichia, genus Serratia, genus Bacillus, genus Brevibacterium, genus Corynebacterium, genus Microbacterium, genus Pseudomonas, genus Synechocystis and the like. Specifically, examples thereof include Escherichia coli, Serratia marsecense, Bacillus subtilis, Bacillus megaterium, Bacillus cereus, Bacillus amyloliquefaciens, Brevibacterium immariophilum, Brevibacterium saccharolyticum, Corynebacterium glutamicum, Corynebacterium ammoniagenes, Microbacterium ammoniaphilum, Pseudomonas putida, Pseudomonas aeruginosa, Synechocystis sp. PCC6803 and the like.

As the DNA which participates in biosynthesis of mevalonic acid from acetyl-CoA herein, any DNA may be used, so long as it encodes an enzyme that is present in a biosynthetic pathway where mevalonic acid can be biosynthesized from acetyl-CoA. Examples of the DNA include a DNA encoding an enzyme in the reaction pathway of the mevalonic acid pathway, from acetyl-CoA until the production of mevalonic acid (hereinafter, referred to as a mevalonic acid biosynthetic enzyme), and the like.

Examples of the mevalonic acid biosynthetic enzyme include an enzyme having the activity to produce acetoacetyl-CoA from acetyl-CoA, an enzyme having the activity to produce 3-hydroxy-3-methylglutaryl coenzyme A (HMG-CoA) from acetoacetyl-CoA, and an enzyme having the activity to produce mevalonic acid from HMG-CoA. Examples of the enzyme having the activity to produce acetoacetyl-CoA from acetyl-CoA include acetyl-CoA acetyltransferase. Examples of the enzyme having the activity to produce HMG-CoA from acetoacetyl-CoA include HMG-CoA synthase. Examples of the enzyme having the activity to produce mevalonic acid from HMG-CoA include HMG-CoA reductase. Further, also included are proteins having two or all three enzyme activities among acetyl-CoA acetyltransferase, HMG-CoA synthase and HMG-CoA reductase in a single enzyme protein, for example, acetyl-CoA acetyltransferase/HMG-CoA reductase having both enzyme activities of acetyl-CoA acetyltransferase and HMG-CoA reductase in a single enzyme.

The DNA encoding the aforementioned enzyme may be derived from any organism and may have an artificial sequence, as long as it functions as a mevalonic acid biosynthetic enzyme in the aforementioned microorganism not having the ability to biosynthesize mevalonic acid from acetyl-CoA when it is expressed in the microorganism as a host, and preferred are DNAs derived from an organism with a codon usage that is similar to the codon usage in the host microorganism, or DNAs with altered sequence such that the codon usages become similar thereto.

Hereinafter, any one of methods described in Molecular Cloning: A Laboratory Manual 3rd ed., Cold Spring Harbor Laboratory Press (2001) (hereinafter, referred to as Molecular Cloning 3rd ed. ) , Current Protocols in Molecular Biology, John Wiley & Sons (1987-2001) (hereinafter, referred to as Current Protocols in Molecular Biology) and the like is applied as a genetic engineering procedure, unless otherwise stated.

### [1] Preparation of a DNA which participates in biosynthesis of mevalonic acid from acetyl-CoA

### (1) Acquisition of nucleotide sequence information of a DNA encoding a mevalonic acid biosynthetic enzyme

A DNA encoding a mevalonic acid biosynthetic enzyme can be prepared by the method mentioned in (2 ) below utilizing known nucleotide sequence information. Nucleotide sequence information of the DNA encoding each enzyme can be acquired by searching for the nucleotide sequence database such as GenBank.

As the DNA encoding the mevalonic acid biosynthetic enzyme which can be used for the preparation of the microorganism having the ability to biosynthesize mevalonic acid of the present invention, and the nucleotide sequence information of which is known, mentioned are genes derived from microorganisms, and the examples are acetyl-CoA acetyltransferase/HMG-CoA reductase gene mvaE and HMG-CoA synthase gene mvaS of Enterococcus faecalis, acetyl-CoA acetyltransferase/HMG-CoA reductase gene mvaE and HMG-CoA synthase gene mvaS of Enterococcus faecium, HMG-CoA reductase gene mvaA and HMG-CoA synthase gene mvaS of Streptococcus pneumoniae, HMG-CoA reductase gene mvaA and HMG-CoA synthase gene mvaS of Streptococcus pyogenes, acetyl-CoA acetyltransferase gene thiL, HMG-CoA synthase gene hmcM and HMG-CoA reductase gene mvaA of Lactococcus lactis, acetyl-CoA acetyltransferase gene mvaC, HMG-CoA synthase gene mvaS and HMG-CoA reductase gene mvaA of Staphylococcus haemolyticus, HMG-CoA synthase gene mvaS and HMG-CoA reductase gene mvaA of Staphylococcus aureus, HMG-CoA synthase gene mvaS and HMG-CoA reductase gene mvaA of Staphylococcus epidermidis, HMG-CoA synthase gene mvaS and HMG-CoA reductase gene mvaA of Borrelia burgdorferi, HMG-CoA synthase gene hmgs and HMG-CoA reductase gene hmgr of Streptomyces griseolosporeus, HMG-CoA reductase gene MTH562 and HMG-CoA synthase gene MTH792 of Methanothermobacter thermoautotrophicum, HMG-CoA reductase gene MJ0705 and HMG-CoA synthase gene MJ1546 of Methanococcus jannaschii, HMG-CoA reductase gene PAB2106 and HMG-CoA synthase gene PAB0906 of Pyrococcus abyssi, HMG-CoA reductase gene PH1805 and HMG-CoA synthase gene PH0677 of Pyrococcus horikoshii, HMG-CoA reductase gene Ta0406 and HMG-CoA synthase gene Ta1455 of Thermoplasma acidophilum, HMG-CoA reductase gene TVN1168 and HMG-CoA synthase gene TVN0130 of Thermoplasma volcanium, and HMG-CoA synthase gene and HMG-CoA reductase gene HMG1 and HMG2 of Saccharomyces cerevisiae.

### (2) Preparation of a DNA encoding a mevalonic acid biosynthetic enzyme

A DNA encoding a mevalonic acid biosynthetic enzyme can be prepared from an organism which has a gene encoding a mevalonic acid biosynthetic enzyme and being found to have a known nucleotide sequence in (1), for example, a bacterium such as that of genus Enterococcus, genus Streptococcus, genus Staphylococcus or genus Lactococcus; a mycobacterium such as that of genus Streptomyces; an archaebacterium such as that of genus Methanothermobacter, genus Methanococcus, genus Pyrococcus or genus Thermoplasma; or a yeast such as that of genus Saccharomyces. Specif ically, the enzyme can be prepared from Enterococcus faecalis, Enterococcus faecium, Streptococcus pneumoniae, Streptococcus pyogenes, Staphylococcus aureus, Staphylococcus epidermidis, Lactococcus lactis, Streptomyces griseolosporeus, Methanothermobacter thermoautotrophicum, Methanococcus jannaschii, Pyrococcus abyssi, Pyrococcus horikoshii, Thermoplasma acidophilum, Thermoplasma volcanium, Saccharomyces cerevisiae or the like.

The aforementioned microorganism is cultured in a medium which contains a carbon source, a nitrogen source, an inorganic salt and the like that can be assimilated by the microorganism, and which permits efficient proliferation of the microorganism, under the condition of the temperature and aeration suitable for growth of the microorganism. After culturing, a chromosomal DNA of the microorganism is isolated and purified according to a known method (for example, a method described in Current Protocols in Molecular Biology).

A region containing a coding region for a mevalonic acid biosynthetic enzyme is appropriately selected from the nucleotide sequence of a chromosomal DNA of the microorganism. A DNA containing at its 3' terminal a sequence of 5'-terminal 20 to 40 bases of the nucleotide sequence of the selected region (forward primer) and a DNA containing at its 3' terminal a complementary sequence to 3'-terminal 20 to 40 bases of the nucleotide sequence of the selected region (reverse primer) are respectively synthesized with a DNA synthesizer. A DNA encoding a mevalonic acid biosynthetic enzyme can be amplified by PCR which is carried out using these two synthetic DNAs as primers, and then can be isolated. PCR can be carried out according to a method described in Molecular Cloning 3rd ed., PCR Protocols, Academic Press (1990). Furthermore, by substituting a nucleotide such that the nucleotide sequence of the coding region for the mevalonic acid biosynthetic enzyme becomes the most suitable codon for the expression in the host microorganism, the expression level of the enzyme can be increased, and consequently, productivity of mevalonic acid can be improved. For example, when the initiation codon is not ATG but GTG, TTG or CTG, the initiation codon of the DNA encoding the mevalonic acid biosynthetic enzyme can be substituted with ATG through PCR using a forward primer in which the sequence corresponding to the initiation codon is substituted with ATG. Moreover, a codon which is rarely used in the host microorganism can be substituted with a codon which is frequently used by site-directed mutagenesis [Nucleic Acids Res., 10, 6487 (1982); Proc. Natl. Acad. Sci. USA, 79, 6409 (1982); Gene, 34, 315 (1985); Nucleic Acids Res., 13, 4431 (1985); Proc. Natl. Acad. Sci. USA, 82, 48 (1985)].

Isolated fragment is inserted into a suitable vector for E. coli as needed, and the nucleotide sequence of the fragment is determined with a DNA sequencer.

### [2] Preparation of a microorganism of the present invention

The microorganism having the ability to biosynthesize mevalonic acid of the present invention is obtainable as a transformant having the activity to produce mevalonic acid from acetyl-CoA by transfecting the DNA encoding the mevalonic acid biosynthetic enzyme prepared in [1] into the host microorganism described below to express the DNA therein.

When the host microorganism does not have a mevalonic acid pathway, the microorganism having the ability to biosynthesize mevalonic acid of the present invention is obtainable by transfecting three types of mevalonic acid biosynthetic enzymes, i.e., acetyl-CoA acetyltransferase, HMG-CoA synthase and HMG-CoA reductase into the microorganism to express the DNAs therein; or by transfecting two types of mevalonic acid biosynthetic enzymes, i.e., acetyl-CoA acetyltransferase/HMG-CoA reductase and HMG-CoA synthase to express the DNAs therein.

When the host microorganism does not have the ability to biosynthesize mevalonic acid from acetyl-CoA, and endogenously has an enzyme having the activity to produce acetoacetyl-CoA from acetyl-CoA, an enzyme having the activity to produce HMG-CoA from acetoacetyl-CoA, an enzyme having the activity to produce mevalonic acid from HMG-CoA, or an enzyme having two activities among these activities in a single enzyme protein, the ability to biosynthesize mevalonic acid can be provided to the host microorganism by transfecting a DNA encoding an enzyme other than the enzyme which the host microorganism has endogenously to express the DNA therein.

When the host microorganism already has the ability to biosynthesize mevalonic acid from acetyl-CoA, the activity of the mevalonic acid biosynthetic enzyme in the host microorganism is enhanced by transfecting a DNA encoding the mevalonic acid biosynthetic enzyme prepared in [1] to express the DNA therein, and consequently, productivity of mevalonic acid can be improved.

### (1) Host microorganism

Although any microorganism can be used as the host microorganism, preferred are microorganisms having the ability to biosynthesize mevalonic acid from acetyl-CoA, microorganisms lacking the ability due to a mutation on the mevalonic acid pathway, or microorganisms having only a non-mevalonic acid pathway. Particularly preferred are microorganisms having only a non-mevalonic acid pathway, because they are believed not to have an activity to metabolize mevalonic acid.

Examples of the microorganism having only a non-mevalonic acid pathway include bacteria belonging to genus Escherichia, genus Serratia, genus Bacillus, genus Brevibacterium, genus Corynebacterium, genus Microbacterium, genus Pseudomonas and genus Synechocystis, and more specifically, include Escherichia coli (hereinafter, abbreviated as E. coli), Serratia marsecense, Bacillus subtilis, Bacillus megaterium, Bacillus cereus, Bacillus amyloliquefaciens, Brevibacterium immariophilum, Brevibacterium saccharolyticum, Corynebacterium glutamicum, Corynebacterium ammoniagenes, Microbacterium ammoniaphilum, Pseudomonas putida, Pseudomonas aeruginosa, Synechocystis sp. PCC6803 and the like.

### (2) A vector containing a DNA encoding a mevalonic acid biosynthetic enzyme

### (a) Utilization of an expression vector

A DNA fragment with a suitable length containing a coding region for a mevalonic acid biosynthetic enzyme is prepared from the DNA encoding a mevalonic acid biosynthetic enzyme prepared by the method mentioned in the above [1]. The DNA fragment is inserted downstream of a promoter of a suitable expression vector to construct a recombinant expression vector.

The expression vector for inserting the DNA encoding a mevalonic acid biosynthetic enzyme is preferably a recombinant DNA which can autonomously replicate in the host microorganism mentioned in (1), and which contains a promoter, a ribosome binding sequence, a restriction enzyme site for inserting the DNA encoding the mevalonic acid biosynthetic enzyme, and a transcription termination sequence. The expression vector may further contain a gene that regulates the promoter. Moreover, it is preferred that the expression vector contains a selection marker gene such as a drug resistant gene for easily selecting the transformant.

Specific examples of the expression vector include plasmids such as pKK223-3 (manufactured by Amersham Biosciences), pGEX-5X-3 (manufactured by Amersham Biosciences), pET-3a (manufactured by Novagen), pGEMEX-1 (manufactured by Promega Corporation), pQE-30 (manufactured by QIAGEN), pKYP10 (Japanese Published Unexamined Patent Application No. 110600/1983), pKYP200 [Agric. Biol. Chem., 48, 669 (1984)], pLSA1 [Agric. Biol. Chem., 53, 277 (1989)], pGEL1 [Proc. Natl. Acad. Sci., USA, 82, 4306 (1985)], pTrS30 [prepared from E. coli JM109/pTrS30 (FERM BP-5407)], pTrS32 [prepared from E. coli JM109/pTrS32 (FERM BP-5408)], pPA1 (Japanese Published Unexamined Patent Application No. 233798/1988), pEG400 [J. Bacteriol., 172, 2392 (1990)], pHW1520 (manufactured by MoBiTec), pRI109 (WO 00/44886) and the like.

The promoter may be any promoter as long as it can initiate the transcription of a gene positioned downstream of the promoter in the host microorganism. Examples thereof include promoters derived from E. coli, phage and the like such as trp promoter, lac promoter, P_{L} promoter, P_{R} promoter and T7 promoter, etc.; SPO1 promoter, SPO2 promoter, penP promoter and xylA promoter for expression in bacterium of genus Bacillus; P54-6 promoter for expression in bacterium of genus Corynebacterium, and the like. In addition, artificially designed and modified promoter such as a promoter in which two trp promoters are linked in tandem, tac promoter, lacT7 promoter or let I promoter can be also used.

It is preferred to use a vector in which the space between Shine-Dalgarno sequence, which is a ribosome binding sequence, and the initiation codon of the DNA encoding the mevalonic acid biosynthetic enzyme is adjusted to an appropriate distance (for example, 6 to 18 nucleotides). A region encoding the mevalonic acid biosynthetic enzyme and a region containing an endogenous ribosome binding sequence that exists upstream of the same in a chromosomal DNA are amplified, which may be used as a ribosome binding sequence in preparing the DNA encoding the mevalonic acid biosynthetic enzyme of [1].

Although a transcription termination sequence is not necessarily required for expression of the mevalonic acid biosynthetic enzyme, it is preferred that a transcription termination sequence is positioned immediately downstream of the DNA encoding the mevalonic acid biosynthetic enzyme (or downstream of the DNA which is positioned most downstream, when two or more DNAs encoding the mevalonic acid biosynthetic enzyme are linked downstream of one promoter).

In order to transfect two or more kinds of DNAs encoding the mevalonic acid biosynthetic enzyme into a host microorganism to express the DNAs therein, a vector which has units for the respective DNAs encoding the mevalonic acid biosynthetic enzyme is constructed, each of the units comprising a promoter, a ribosome binding sequence, a DNA encoding a mevalonic acid biosynthetic enzyme and a transcription termination sequence; or alternatively constructed is a vector in which units for the respective DNAs are serially linked downstream of one promoter, and a transcription termination sequence is present downstream of the last unit, each of the units comprising a ribosome binding sequence and a DNA encoding a mevalonic acid biosynthetic enzyme.

Examples of the expression vector containing a DNA encoding a mevalonic acid biosynthetic enzyme constructed as described above include pMV1 containing an acetyl-CoA acetyltransferase/HMG-CoA reductase gene mvaE of Enterococcus faecalis, pMV2 containing mvaE and an HMG-CoA synthase gene mvas of Enterococcus faecalis, pMV4 containing an acetyl-CoA acetyltransferase gene thiL and an HMG-CoA reductase gene mvaA of Lactococcus lactis, and pMV5 containing thiL, mvaA and an HMG-CoA synthase gene hmcM of Lactococcus lactis.

### (b) Expression by endogenous promoter

When a region encoding the mevalonic acid biosynthetic enzyme in the chromosomal DNA together with a region containing an endogenous ribosome binding sequence and a promoter that are present upstream thereof are amplified in preparing the DNA encoding the mevalonic acid biosynthetic enzyme of [1], the mevalonic acid biosynthetic enzyme can be expressed without using the abovementioned expression vector by means of the endogenous promoter by transfecting the DNA into a host microorganism. For the transfection, a recombinant vector is constructed by inserting the DNA into a vector which can autonomously replicate in a host microorganism and contains a restriction enzyme site for inserting the DNA and a selection marker gene such as a drug resistant gene for easily selecting the transformant. Examples of the vector which may be used for inserting the DNA include pBR322 [Gene, 2, 95 (1977)], pBluescript II KS (+) (manufactured by Stratagene), pUC18 [Gene, 33, 103 (1985)], pHY300PLK (manufactured by Takara Shuzo Co., Ltd.), and the like.

### (3) Transfection of a vector containing a DNA encoding mevalonic acid biosynthetic enzyme into a host microorganism, and preparation of a transformant

As a method for transfecting the vector containing the DNA encoding a mevalonic acid biosynthetic enzyme constructed in (2) into a host microorganism, any method for transfecting a DNA into a host microorganism mentioned in (1) can be used, and examples thereof include a method in which a calcium ion is used [Proc. Natl. Acad. Sci., USA, 69, 2110 (1972)], a protoplast method (Japanese Published unexamined Patent Application No. 248394/1988), an electroporation method [Nucleic Acids Research, 16, 6127 (1988)], and the like. The host microorganism transfected with the vector is cultured, and the transformant is obtainable through selecting on the basis of the change of the phenotype (resistance against a drug, or the like) according to the selection marker in the expression vector.

Examples of the recombinant microorganism having the ability to biosynthesize mevalonic acid prepared as described hereinabove include E. coli strain XL1-Blue/pMV2 which is prepared by transfecting pMV2, an expression vector of acetyl-CoA acetyltransferase/HMG-CoA reductase and HMG-CoA synthase, into E. coli XL1-Blue, and E. coli strain NM522/pMV5 which is prepared by transfecting pMV5, an expression vector of acetyl-CoA acetyltransferase, HMG-CoA reductase and HMG-CoA synthase, into E. coli NM552.

### [3] Production of mevalonic acid

### (1) Culture of a recombinant microorganism

Medium for culturing the recombinant microorganism obtainable by the method described in [2] above may be either a natural medium or a synthetic medium, as long as it is a medium which contains a carbon source, a nitrogen source, an inorganic salt and the like that can be assimilated by the microorganism, and which permits efficient culture of the microorganism.

The carbon source may be any carbon source which can be assimilated by the microorganism, and examples thereof include carbohydrates such as glucose, fructose, sucrose, molasses containing the same and starch or starch hydrolysate, organic acids such as acetic acid and propionic acid, alcohols such as ethanol and propanol, and the like.

Examples of the nitrogen source include ammonium salts of an inorganic acid or organic acid such as ammonia, ammonium chloride, ammonium sulfate, ammonium acetate and ammonium phosphate, other nitrogenous compounds, peptone, meat extract, yeast extract, corn steep liquor, casein hydrolysate, soy bean cake and soy bean cake hydrolysate, microbial body used for the fermentation and the digestion product of the same, and the like.

Examples of the inorganic salt include potassium dihydrogenphosphate, dipotassium hydrogenphosphate, magnesium phosphate, magnesium sulfate, sodium chloride, ferrous sulfate, manganese sulfate, copper sulfate, calcium carbonate and the like.

An antibiotic such as ampicillin or tetracycline may be added to the medium, if necessary. Further, when a microorganism transformed with an expression vector containing an inducible promoter is cultured, the inducer for the promoter may be added to the medium during the cultivation, if necessary. Examples of the inducer include isopropyl-β-D-thiogalactoside for lac promoter, indoleacrylic acid for trp promoter, and the like.

Cultivation is usually conducted under an aerobic condition , by shaking culture or aerating with stirring culture. The cultivation temperature is preferably 15 to 40°C, and the cultivation time is usually 5 hours to 7 days. During the cultivation, pH is kept at 3.0 to 9.0. The pH is adjusted using an inorganic acid or an organic acid, an alkaline solution, urea, calcium carbonate, aqueous ammonia or the like.

### (2) Recovery of mevalonic acid from culture

Mevalonic acid can be recovered from the culture medium using an isolation and purification method which is generally employed in organic chemistry such as extraction with an organic solvent, crystallization, thin layer chromatography and high performance liquid chromatography.

The recovered mevalonic acid can be subjected to structural verification or quantitative determination by ¹³C-NMR spectrum, ¹H-NMR spectrum, mass spectrum, gas chromatography, high performance liquid chromatography or the like.

Examples of the present invention are shown below, however the present invention is not limited to these Examples.

### Brief Description of the Drawings

Fig. 1 shows a construction step of pMV1.
Fig. 2 shows a construction step of pMV2.
Fig. 3 shows a construction step of pMV4.
Fig. 4 shows a construction step of pMV5.

### [Description of Abbreviation]

Ampr:ampicillin resistant gene
Ptrp:trp promoter
mvaE: acetyl-CoA acetyltransferase/HMG-CoA reductase gene
mvaS:HMG-CoA synthase gene
thiL:acetyl-CoA acetyltransferase gene
mvaA:HMG-CoA reductase gene
hmcM:HMG-CoA synthase gene

### Best Mode for Carrying Out the Invention

### [Example 1] Search for genes on database

Using acetyl-coenzyme A acetyltransferase, 3-hydroxy-3-methylglutaryl-coenzyme A synthase and 3-hydroxy-3-methylglutaryl coenzyme A reductase as a query, key word search was carried out on the nucleotide sequence database GenBank.

As a consequence, sequence information was obtained as to the following enzyme genes derived from the bacteria, i.e., acetyl-CoA acetyltransferase/HMG-CoA reductase gene mvaE (GenBank Accession No.: AF290092) and HMG-CoA synthase gene mvaS (GenBank Accession No.: AF290092) of Enterococcus faecalis, acetyl-CoA acetyltransferase/HMG-CoA reductase gene mvaE (GenBank Accession No.: AF290094) and HMG-CoA synthase gene mvaS (GenBank Accession No.: AF290094) of Enterococcus faecium, HMG-CoA reductase gene mvaA (GenBank Accession No.: AF290098) and HMG-CoA synthase gene mvaS (GenBank Accession No.: AF290098) of Streptococcus pneumoniae, HMG-CoA reductase gene mvaA (GenBank Accession No.: AF290096) and HMG-CoA synthase gene mvaS (GenBank Accession No.: AF290096) of Streptococcus pyogenes, acetyl-CoA acetyltransferase gene thiL (GenBank Accession No.: AE006387), HMG-CoA synthase gene hmcM (GenBank Accession No.: AE006388) and HMG-CoA reductase gene mvaA (GenBank Accession No.: AE006387) of Lactococcus lactis, acetyl-CoA acetyltransferase gene mvaC (GenBank Accession No.: AF290088), HMG-CoA synthase gene mvaS (GenBank Accession No.: AF290088) and HMG-CoA reductase gene mvaA (GenBank Accession No.: AF290088) of Staphylococcus haemolyticus, HMG-CoA synthase gene mvaS (GenBank Accession No.: AF290086) and HMG-CoA reductase gene mvaA (GenBank Accession No.: AF290086) of Staphylococcus aureus, HMG-CoA synthase gene mvaS (GenBank Accession No.: AF290090) and HMG-CoA reductase gene mvaA (GenBank Accession No.: AF290090) of Staphylococcus epidermidis, HMG-CoA synthase gene BB0683 (GenBank Accession No.: AE001169) and HMG-CoA reductase gene BB0685 (GenBank Accession No.: AE001169) of Borrelia burgdorferi and the like; as to the following enzyme genes derived from the mycobacteria, i.e., HMG-CoA synthase gene hmgs (GenBank Accession No.: AB037907) and HMG-CoA reductase gene hmgr (GenBank Accession No.: AB037907) of Streptomyces griseolosporeus and the like; as to the following enzyme genes derived from the archaebacteria, i.e., HMG-CoA reductase gene MTH562 (GenBank Accession No.: NC_000916) and HMG-CoA synthase gene MTH792 (GenBank Accession No.: NC_000916) of Methanothermobacter thermoautotrophicum, HMG-CoA reductase gene MJ0705 (GenBank Accession No.: NC_000909) and HMG-CoA synthase gene MJ1546 (GenBank Accession No.: NC_000909) of Methanococcus jannaschii, HMG-CoA reductase gene PAB2106 (GenBank Accession No.: NC_000868) and HMG-CoA synthase gene PAB0906 (GenBank Accession No.: NC_000868) of Pyrococcus abyssi, HMG-CoA reductase gene PH1805 (GenBank Accession No.: NC_000961) and HMG-CoA synthase gene PH0677 (GenBank Accession No.: NC_000961) of Pyrococcus horikoshii, HMG-CoA reductase gene Ta0406 (GenBank Accession No. : AL445064) and HMG-CoA synthase gene Ta1455 (GenBank Accession No.: NC_002578) of Thermoplasma acidophilum, HMG-CoA reductase gene TVN1168 (GenBank Accession No.: NC_002689) and HMG-CoA synthase gene TVN0130 (GenBank Accession No.: NC_002689) of Thermoplasma volcanium and the like; as to the following enzyme genes derived from the yeast, i.e., HMG-CoA synthase gene (GenBank Accession No.: X96617) and HMG-CoA reductase gene HMG1 (GenBank Accession No. : NC_001145) and HMG2 (GenBank Accession No.: NC_001144) of Saccharomyces cerevisiae and the like; and as to the enzyme genes derived from higher eukaryote such as mouse, human or Arabidopsis thaliana.

### [Example 2] Preparation of E. coli strain that expresses mevalonic acid biosynthetic enzyme genes derived from Enterococcus faecalis

### (1) Isolation of mvaE and mvaS derived from Enterococcus faecalis

Among the sequence information obtained in Example 1, a sequence (GenBank Accession No.: AF290092) containing the gene mvaE encoding acetyl-CoA acetyltransferase/HMG-CoA reductase and the gene mvaS encoding HMG-CoA synthase of Enterococcus faecalis (hereinafter, abbreviated as E. faecalis) was utilized to obtain the gene fragments as described below. A nucleotide sequence of mvaE of E. faecalis (hereinafter, merely described as mvaE) is shown in SEQ ID NO: 1; an amino acid sequence of acetyl-CoA acetyltransferase/HMG-CoA reductase encoded by the gene is shown in SEQ ID NO: 2; a nucleotide sequence of mvaS of E. faecalis (hereinafter, merely described as mvaS) is shown in SEQ ID NO: 3; and an amino acid sequence of HMG-CoA synthase encoded by the gene is shown in SEQ ID NO: 4.

First, E. faecalis (ATCC 14508) was inoculated into a brain heart infusion broth (manufactured by Difco), and subjected to static culture overnight at 30°C. After the cultivation, a chromosomal DNA of the microorganism was isolated and purified according to a method in which saturated phenol is used, as described in Current Protocols in Molecular Biology.

Using 8905 DNA synthesizer manufactured by Perseptive Biosystems, Inc., DNAs having the sequences shown in SEQ ID NO: 5 to 8, respectively, (hereinafter, referred to as primer A, primer B, primer C, primer D, respectively) were synthesized. The sequence of SEQ ID NO: 5 corresponds to a sequence of positions 1 to 24 of the sequence of SEQ ID NO: 9 (partial sequence of the chromosomal DNA of E. faecalis containing MvaE), in which the initiation codon (ttg) of MvaE is substituted with atg, and a sequence containing a ClaI recognition sequence is added at its 5' terminal. The sequence of SEQ ID NO: 6 corresponds to a sequence complementary to the sequence of positions 2395 to 2418 of the sequence of SEQ ID NO: 9, in which a sequence containing a BamHI recognition sequence is added at its 5' terminal. The sequence of SEQ ID NO: 7 corresponds to a sequence of positions 1 to 20 of the sequence of SEQ ID NO: 10 (partial sequence of the chromosomal DNA of E. faecalis containing MvaS), in which a sequence containing a BglII recognition sequence is added at its 5' terminal. The sequence of SEQ ID NO: 8 corresponds to a sequence complementary to the sequence of positions 1144 to 1166 of the sequence of SEQ ID NO: 10, in which a sequence containing a BamHI recognition sequence is added at its 5' terminal.

Using the chromosomal DNA of E. faecalis as a template, PCR was conducted using the aforementioned primer A and primer B for amplification of the mvaE fragment, or the primer C and primer D for amplification of the mvaS fragment, respectively. PCR was conducted by subjecting 40 µL of a reaction solution containing 0.1 µg of the chromosomal DNA, 0.5 µmol/L of each primer, 2.5 units of Pfu DNA polymerase (manufactured by Stratagene), 4 µL of x 10 buffer for Pfu DNA polymerase (manufactured by Stratagene) and 200 µmol/L of each dNTP (dATP, dGTP, dCTP and TTP) to the cycles of at 94°C for 1 min, at 55°C for 2 min and at 72°C for 3 min repeated 30 times.

Aliquot of each reaction solution in an amount of 1/10 was subjected to an agarose gel electrophoresis, and after the confirmation that a fragment of about 2.4 kb corresponding to the mvaE fragment was amplified by PCR using primer A and primer B and that a fragment of about 1.1 kb corresponding to the mvaS fragment was amplified by PCR using primer C and primer D, each residual reaction solution was mixed with an equal volume of TE [10 mmol/L Tris-HCl (pH8.0), 1 mmol/L EDTA] saturated phenol/chloroform (1 vol/1 vol). Each mixture was centrifuged, and the resulting upper layer was mixed with two fold volume of cold ethanol. Each mixture was left to stand at -80°C for 30 min, and centrifuged to give a precipitate of the DNA. The precipitate of each DNA was dissolved in 20 µL of TE.

Using each 5 µL of the solution, the DNA amplified with primer A and primer B was cleaved with restriction enzymes ClaI and BamHI, while the DNA amplified with primer C and primer D was cleaved with restriction enzymes BglII and BamHI . After separating the DNA fragments by agarose gel electrophoresis, DNA fragments of 2.4 kb containing MvaE and of 1.1 kb containing MvaS were recovered, respectively, with GENECLEAN II kit, (manufactured by BIO 101, Inc.).

### (2) Construction of an expression vector pMV1 containing MvaE

After 0.2 µg of an expression vector pTrs30 [which can be prepared from E. coli JM109/pTrS30 (FERM BP-5407)] containing a trp promoter was cleaved with restriction enzymes ClaI and BamHI, the DNA fragments were separated by agarose gel electrophoresis, and a DNA fragment of 4.6 kb was similarly recovered.

Using the fragment of 2.4 kb containing MvaE prepared in (1) and the fragment of 4.6 kb, and a ligation kit (manufactured by Takara Shuzo Co., Ltd.), a ligation reaction was conducted at 16°C for 16 hrs.

E. coli strain XL1-Blue (manufactured by Stratagene) was transformed with the ligation reaction solution according to the method in which a calcium ion is used [Proc. Natl. Acad. Sci., USA, 69, 2110 (1972)]. The transformant was inoculated on an LB agar medium (10 g/L triptone, 5 g/L yeast extract, 10 g/L sodium chloride, 15 g/L agar, pH 7.0) containing 50 µg/mL ampicillin, and cultured at 30°C overnight.

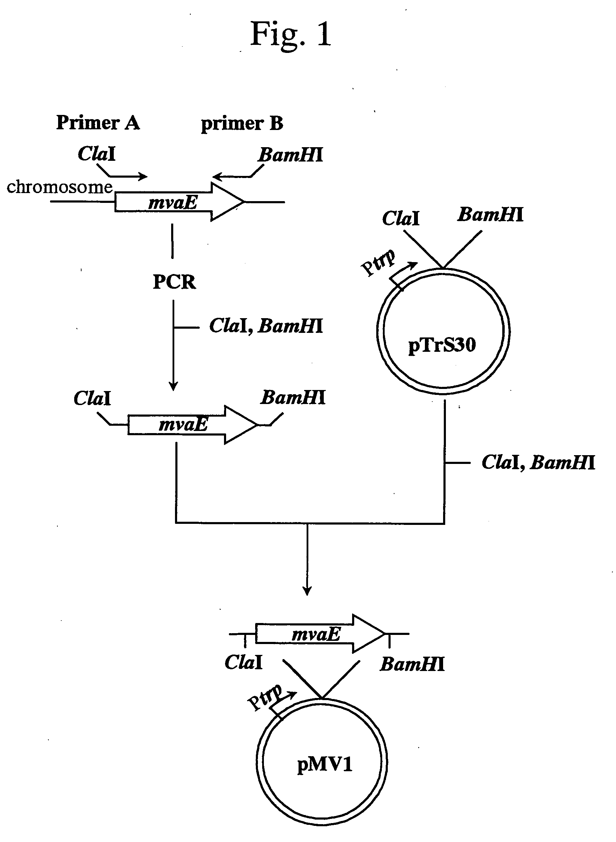

Plasmid was extracted from the colony of the grown transformant according to a known method to obtain pMV1 that is an expression vector of acetyl-CoA acetyltransferase/HMG-CoA reductase containing mvaE. Structure of the vector was determined by restriction enzyme digestion. The construction step as described hereinabove is illustrated in Fig. 1.

### (3) Construction of an expression vector pMV2 containing each gene fragment of MvaE and MvaS

After cleaving pMV1 with a restriction enzyme BamHI, the DNA fragments were separated by agarose gel electrophoresis, and a DNA fragment of 7 kb was recovered with GENECLEAN II kit. Dephosphorylation of ends of the DNA fragment was carried out with a blunt DNA kit (manufactured by Takara Shuzo Co., Ltd.).

Using the fragment of 7 kb and the fragment of 1.1 kb containing MvaS, and the ligation kit, a ligation reaction was conducted at 16°C for 16 hrs.

E. coli strain XL1-Blue was transformed with the ligation reaction solution according to the aforementioned method. The transformant was inoculated on an LB agar medium containing 50 µg/mL ampicillin, and cultured at 30°C overnight.

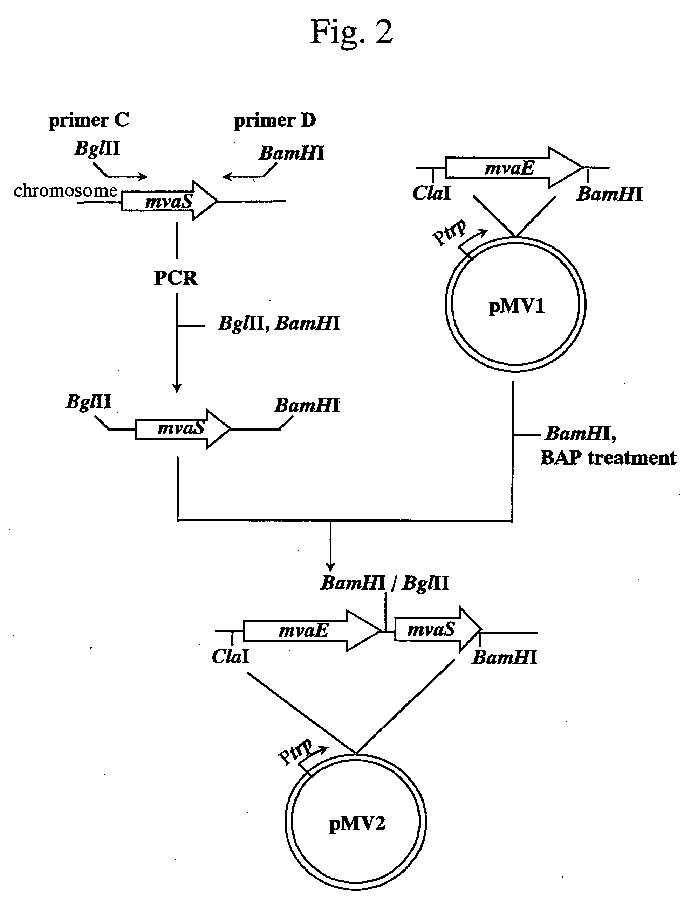

Plasmid was extracted from the colony of the grown transformant according to the aforementioned method, and the structure of the plasmid was determined by restriction enzyme digestion. As a result, it was confirmed that pMV2, an expression vector having a structure with MvaE and MvaS inserted in a forward direction of their transcription from the trp promoter, was obtained. The construction step as described hereinabove is illustrated in Fig. 2. pMV2 is an expression vector of acetyl-CoA acetyltransferase/HMG-CoA reductase and HMG-CoA synthase. The transformant containing pMV2 is designated as E. coli strain XL1-Blue/pMV2.

### [Example 3] Production of mevalonic acid by E. coli strain XL1-Blue/pMV2

### (1) Production of mevalonic acid by culturing in a test tube

The E. coli strain XL1-Blue/pMV2 obtained in Example 2 was inoculated into 8 mL of an LB medium (10 g/L triptone, 5 g/L yeast extract, 10 g/L sodium chloride, pH 7.0) containing 50 µg/mL ampicillin in a large test tube, and cultured at 28°C for 17 hrs. The culture medium was inoculated into 8 mL of an M9X medium [16 g/L dipotassium hydrogenphosphate, 14 g/L potassium dihydrogenphosphate, 5 g/L ammonium sulfate, 1 g/L citric acid (anhydride), 5 g/L peptone (manufactured by Kyokuto Pharmaceutical Industrial Co., Ltd. ), 20 g/L glucose, 0. 8 mg/L vitamin B₁, 25 mg/L magnesium sulfate heptahydrate, 50 mg/L ferrous sulfate heptahydrate, pH 7.2, adjusted with 10 mol/L sodium hydroxide: Glucose, vitamin B₁, magnesium sulfate heptahydrate and ferrous sulfate heptahydrate were separately added after autoclaving] containing 100 µg/mL ampicillin in a large test tube at 1%, and cultured at 30°C for 48 hrs. The culture medium was centrifuged to prepare the culture supernatant. As a control, a transformant E. coli strain XL1-Blue/pTrS30 which is a transformant containing a vector pTrS30 was similarly cultured, and the culture supernatant was prepared.

The culture product in each culture supernatant was analyzed with gas chromatography according to the method described in Agr. Biol. Chem. 37, 1013 (1973). It was confirmed that 3.9 g/L mevalonic acid was produced and accumulated in the culture medium of the E. coli strain XL1-Blue/pMV2. On the other hand, production of mevalonic acid was not found in the E. coli strain XL1-Blue/pTrS30 containing the vector pTrS30 alone.

### (2) Production of mevalonic acid using a 2 L jar fermenter

The E. coli strain XL1-Blue/pMV2 obtained in Example 2 was inoculated into 50 mL of an LB medium containing 100 µg/mL ampicillin in an Erlenmeyer flask, and cultured at 28°C for 17 hrs.

The culture medium was inoculated into 950 mL of an M9XA medium [7 g/L potassium dihydrogenphosphate, 5 g/L ammonium chloride, 1 g/L citric acid (anhydride), 1 g/L peptone (manufactured by Kyokuto Pharmaceutical Industrial Co., Ltd.), 10 mg/L manganese sulfate heptahydrate, 20 g/L glucose, 10 mg/L vitamin B₁, 2 g/L magnesium sulfate heptahydrate, 0.2 g/L ferrous sulfate heptahydrate, pH 7.2, adjusted with 10 mol/L sodium hydroxide: Glucose, vitamin B₁, magnesium sulfate heptahydrate and ferrous sulfate heptahydrate were separately added after autoclaving] in a 2 L jar fermenter at 1%. During the cultivation, pH of the medium was kept at pH 7.0 with 18% aqueous ammonia. Culturing was conducted by stirring at 900 rpm and aeration with compressed air at 1.0 vvm which was sterilized via a filter for sterilization. Culturing was conducted at a temperature of 30°C. After depletion of the initially added sugar (after 24 hours), a sterilized 60% glucose solution was supplied at a rate of 10 to 13 mL per hour.

The culture product in the culture supernatant was analyzed with gas chromatography, and it was confirmed that 50.2 g/L mevalonic acid was produced and accumulated in the culture medium after 50 hours of cultivation.

### [Example 4] Preparation of E. coli strain that expresses mevalonic acid biosynthetic enzymes derived from Lactococcus lactis

### (1) Isolation of DNA fragments containing of thiL gene, mvaA gene and hmcM gene respectively derived from Lactococcus lactis

Among the sequence information obtained in Example 1, the sequence (GenBank Accession No.: AE006387) containing the acetyl-CoA acetyltransferase gene thiL and HMG-CoA reductase gene mvaA of Lactococcus lactis (hereinafter, abbreviated as L. lactis), and the sequence (GenBank Accession No. : AE0O6388) containing the HMG-CoA synthase gene hmcM of L. lactis were utilized to prepare these gene fragments as follows.

A nucleotide sequence of thiL of L. lactis (hereinafter, merely described as thiL) is shown in SEQ ID NO: 11; an amino acid sequence of acetyl-CoA acetyltransferase encoded by the gene is shown in SEQ ID NO: 12; a nucleotide sequence of mvaA of L. lactis (hereinafter, merely described as mvaA) is shown in SEQ ID NO: 13; an amino acid sequence of HMG-CoA reductase encoded by the gene is shown in SEQ ID NO: 14; hmcM of L. lactis (hereinafter, merely described as hmcM) is shown in SEQ ID NO: 15; and an amino acid sequence of HMG-CoA synthase encoded by the gene is shown in SEQ ID NO: 16.

L. lactis (ATCC No. 13675) was inoculated into a brain heart infusion broth (manufactured by Difco), and subjected to static culture overnight at 30°C. After the cultivation, a chromosomal DNA of the microorganism was isolated and purified according to a method in which saturated phenol is used, as described in Current Protocols in Molecular Biology.

Using 8905 DNA synthesizer manufactured by Perseptive Biosystems, Inc., DNAs having the sequences of SEQ ID NO: 17 to 20, respectively, (hereinafter, referred to as primer E, primer F, primer G, primer H, respectively) were synthesized.

The sequence of SEQ ID NO: 17 corresponds to a sequence of positions 1 to 21 of the sequence of SEQ ID NO: 21 (partial sequence of the chromosomal DNA of L lactis containing thiL and MvaA), in which the initiation codon (gtg) of thiL is substituted with atg and a sequence containing a ClaI recognition sequence is added at its 5' terminal. The sequence of SEQ ID NO: 18 corresponds to a sequence complementary to the sequence of positions 2444 to 2464 of the sequence of SEQ ID NO: 21, in which a sequence containing a BamHI recognition sequence is added at its 5' terminal. The sequence of SEQ ID NO: 19 corresponds to a sequence of positions 1 to 17 of the sequence of SEQ ID NO: 22 (partial sequence of the chromosomal DNA of L lactis containing hmcM), in which a sequence containing a BglII recognition sequence is added at its 5' terminal. The sequence of SEQ ID NO: 20 corresponds to a sequence complementary to the sequence of positions 1159 to 1179 of the sequence of SEQ ID NO: 22, in which a sequence containing a BamHI recognition sequence is added at its 5' terminal.

Using the chromosomal DNA of L. lactis as a template, PCR was conducted using the aforementioned primer E and primer F for amplification of thiL and mvaA, and using primer G and primer H for amplification of hmcM, respectively. PCR was conducted by subjecting 40 µL of a reaction solution containing 0.1 µg of the chromosomal DNA, 0.5 µmol/L of each primer, 2.5 units of Pfu DNA polymerase (manufactured by Stratagene), 4 µL of x 10 buffer for Pfu DNA polymerase (manufactured by Stratagene) and 200 µmol/L of each dNTP to the cycles of at 94°C for 1 min, at 55°C for 2 min and at 72°C for 3 min repeated 30 times.

Aliquot of each reaction solution in an amount of 1/10 was subjected to on an agarose gel electrophoresis, and after the confirmation that a fragment of about 2.5 kb containing thiL and mvaA was amplified by PCR using primer E and primer F and that a fragment of about 1.2 kb containing hmcM was amplified by PCR using primer G and primer H, each residual reaction mixture was mixed with an equal volume of TE saturated phenol/chloroform. Each mixture was centrifuged, and the resulting upper layer was mixed with two-fold volume of cold ethanol. Each mixture was left to stand at -80°C for 30 min, and centrifuged to give a precipitate of the DNA. The precipitate of each DNA was dissolved in 20 µL of TE.

Using 5 µL of the solution, the DNA amplified with primer E and primer F was cleaved with restriction enzymes ClaI and BamHI, while the DNA amplified with primer G and primer H was cleaved with restriction enzymes BglII and BamHI. After separating the DNA fragments by agarose gel electrophoresis, DNA fragments of 2.5 kb containing thiL and mvaA and of 1.2 kb containing hmcM were recovered, respectively, with GENECLEAN II kit.

### (2) Construction of an expression vector pMV4 containing thiL and mvaA

Using the fragment of 2.5 kb containing thiL and mvaA prepared in (1) and the ClaI-BamHI fragment (4.6 kb) of pTrS30 prepared in Example 2(2), and a ligation kit (manufactured by Takara Shuzo Co., Ltd.), a ligation reaction was conducted at 16°C for 16 hrs.

E. coli strain NM522 [ATCC No. 47000, J. M. Biol. , 166, 1 (1983) ] was transformed with the ligation reaction solution according to the method described above. The transformant was inoculated on an LB agar medium containing 50 µg/mL ampicillin, and cultured at 30°C overnight.

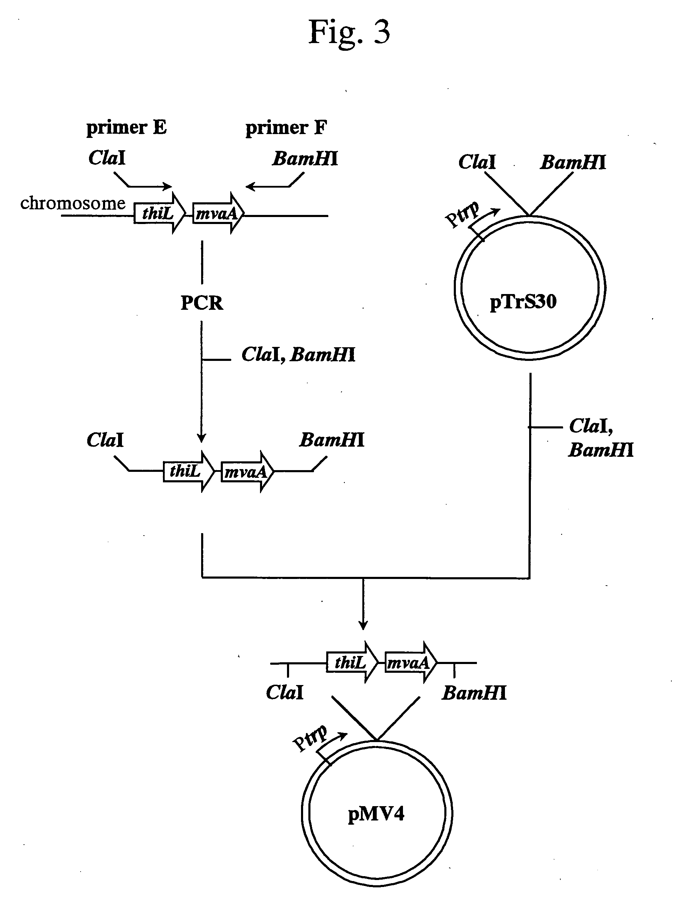

Plasmid was extracted from the colony of the grown transformant according to the method described above to obtain pMV4 that is an expression vector containing thiL and mvaA. pMV4 is an expression vector of acetyl-CoA acetyltransferase and HMG-CoA reductase. Structure of the vector was determined by restriction enzyme digestion. The construction step as described hereinabove is illustrated in Fig. 3.

### (3) Construction of an expression vector pMV5 containing thiL, mvaA and hmcM

After cleaving pMV4 with a restriction enzyme BamHI, the DNA fragments were separated by agarose gel electrophoresis, and a DNA fragment of 7 kb was recovered with GENECLEAN II kit. Dephosphorylation of ends of the fragment was carried out with a blunt DNA kit (manufactured by Takara Shuzo Co., Ltd.). Using the fragment of 7 kb and the fragment of 1.2 kb containing hmcM prepared in (1), and the ligation kit, a ligation reaction was conducted at 16°C for 16 hrs.

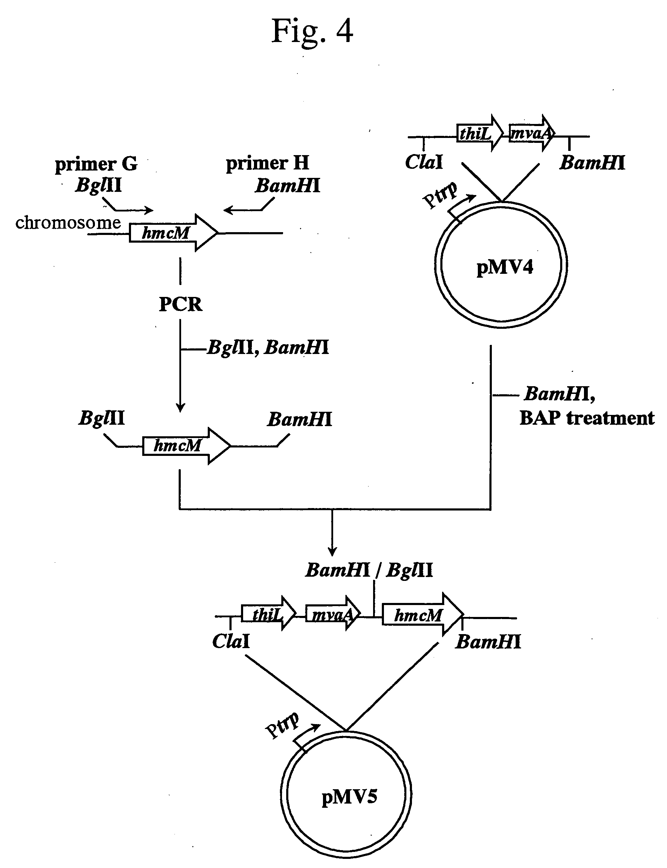

E. coli strain NM522 was transformed with the ligation reaction solution according to the aforementioned method. The transformant was inoculated on an LB agar medium containing 50 µg/mL ampicillin, and cultured at 30°C overnight. Plasmid was extracted from the colony of the grown transformant according to the aforementioned known method, and the structure of the plasmid was determined by restriction enzyme digestion. As a result, it was confirmed that pMV5, an expression vector having a structure with thiL, mvaA and hmcC inserted in a forward direction of their transcription from the trp promoter, was obtained. The construction step as described hereinabove is illustrated in Fig. 4. pMV5 is an expression vector of acetyl-CoA acetyltransferase, HMG-CoA reductase and HMG-CoA synthase. The transformant containing pMV5 is designated as E. coli strain NM522/pMV5.

### [Example 5] Production of mevalonic acid by E. coli strain NM522/pMV5

### (1) Production of mevalonic acid by culturing in a test tube

The E. coli strain NM522/pMV5 obtained in Example 4 was inoculated into 8 mL of an LB medium containing 50 µg/mL ampicillin in a large test tube, and cultured at 28°C for 17 hrs. The culture medium was inoculated into 8 mL of an M9X medium containing 100 µg/mL ampicillin in a large test tube at 1%, and cultured at 30°C for 48 hrs. The culture medium was centrifuged to prepare the culture supernatant. As a control, a transformant E. coli NM522/pTrS30 strain containing a vector pTrS30 was similarly cultured to prepare the culture supernatant.

The culture product in each culture supernatant was analyzed with gas chromatography according to the method described above. It was confirmed that 1.0 g/L mevalonic acid was produced and accumulated in the culture fluid. On the other hand, production of mevalonic acid was not found in the E. coli strain NM522/pTrS30 containing the vector pTrS30 alone.

### (2) Production of mevalonic acid using a 2 L jar fermenter

The E. coli strain NM522/pMV5 obtained in Example 4 was inoculated into 50 mL of an LB medium containing 100 µg/mL ampicillin in an Erlenmeyer flask, and cultured at 28°C for 17 hrs.

The culture fluid was inoculated into 950 mL of an M9XA medium in a 2 L jar fermenter at 1%. During the cultivation, pH of the medium was kept at pH 7.0 with 18% aqueous ammonia. Culturing was conducted by stirring at 900 rpm and aeration with compressed air at 1.0 vvm which was sterilized via a filter for sterilization. Culturing was conducted at a temperature of 30°C. After depletion of the initially added sugar (after 24 hours), a sterilized 60% glucose solution was supplied at a rate of 10 to 13 mL per hour.

The culture product in the culture supernatant was analyzed with gas chromatography, and it was confirmed that 15.3 g/L mevalonic acid was produced and accumulated in the culture medium after 50 hours of cultivation.

### Industrial Applicability

According to the present invention, mevalonic acid can be efficiently produced from an inexpensive raw material.

### [Sequence Listing Free Text]

SEQ ID NO: 1 - inventor: Tabata, Kazuhiko; Hashimoto, Shinichi
SEQ ID NO: 5 - primer A
SEQ ID NO: 6 - primer B
SEQ ID NO: 7 - primer C
SEQ ID NO: 8 - primer D
SEQ ID NO: 17 - primer E
SEQ ID NO: 18 - primer F
SEQ ID NO: 19 - primer G
SEQ ID NO: 20 - primer H

## Claims

1. A process for producing mevalonic acid, which comprises:
culturing a microorganism having the ability to biosynthesize mevalonic acid from acetyl coenzyme A (acetyl-CoA) in a medium, the microorganism being obtainable by transfecting a DNA participating in biosynthesis of mevalonic acid from acetyl-CoA into a host microorganism;
allowing production and accumulation of mevalonic acid in the culture; and
recovering mevalonic acid from the culture.

2. The process according to claim 1, wherein the host microorganism is a microorganism not having the ability to biosynthesize mevalonic acid from acetyl-CoA.

3. The process according to claim 2, wherein the microorganism not having the ability to biosynthesize mevalonic acid from acetyl-CoA is a microorganism not having a mevalonic acid pathway.

4. The process according to any one of claims 1 to 3, wherein the DNA to be transfected is one or more DNA(s) selected from the group consisting of a DNA encoding an enzyme having the activity to produce acetoacetyl-CoA from acetyl-CoA, a DNA encoding an enzyme having the activity to produce 3-hydroxy-3-methylglutaryl coenzyme A (HMG-CoA) from acetoacetyl-CoA, and a DNA encoding an enzyme having the activity to produce mevalonic acid from HMG-CoA.

5. The process according to claim 4, wherein the DNA encoding an enzyme having the activity to produce acetoacetyl-CoA from acetyl-CoA is acetyl-CoA acetyltransferase.

6. The process according to claim 4, wherein the DNA encoding an enzyme having the activity to produce HMG-CoA from acetoacetyl-CoA is HMG-CoA synthase.

7. The process according to claim 4, wherein the DNA encoding an enzyme having the activity to produce mevalonic acid from HMG-CoA is HMG-CoA reductase.

8. The process according to claim 4, wherein the DNA encoding an enzyme having the activity to produce acetoacetyl-CoA from acetyl-CoA and the DNA encoding an enzyme having the activity to produce mevalonic acid from HMG-CoA are a DNA encoding acetyl-CoA acetyltransferase/HMG-CoA reductase.

9. The process according to any one of claims 1 to 3, wherein the DNA to be transfected is a DNA encoding acetyl-CoA acetyltransferase, a DNA encoding HMG-CoA synthase and a DNA encoding HMG-CoA reductase.

10. The process according to any one of claims 1 to 3, wherein the DNA to be transfected is a DNA encoding acetyl-CoA acetyltransferase/HMG-CoA reductase and HMG-CoA synthase.

11. The process according to any one of claims 1 to 10, wherein the DNA is derived from one or more microorganism( s ) selected from the group consisting of bacteria, mycobacteria, archaebacteria and yeast.

12. The process according to any one of claims 1 to 10, wherein the DNA is derived from a microorganism belonging to a genus selected from the group consisting of genus Enterococcus, genus Streptococcus, genus Staphylococcus, genus Lactococcus, genus Streptomyces, genus Methanothermobacter, genus Methanococcus, genus Pyrococcus, genus Thermoplasma and genus Saccharomyces.

13. The process according to any one of claims 1 to 3, wherein the DNA to be transfected is one or more DNA(s) selected from the group consisting of the following (a) to (j):
(a) mvaE of Enterococcus faecalis;
(b) mvaS of Enterococcus faecalis;
(c) thiL of Lactococcus lactis;
(d) mvaA of Lactococcus lactis;
(e) hmcC of Lactococcus lactis;
(f) a DNA encoding an amino acid sequence of SEQ ID NO: 2;
(g) a DNA encoding an amino acid sequence of SEQ ID NO: 4;
(h) a DNA encoding an amino acid sequence of SEQ ID NO: 12;
(i) a DNA encoding an amino acid sequence of SEQ ID NO: 14; and
(j) a DNA encoding an amino acid sequence of SEQ ID NO: 16.

14. T̂he process according to claim 3, wherein the microorganism not having a mevalonic acid pathway is a microorganism belonging to a genus selected from the group consisting of genus Escherichia, genus Serratia, genus Bacillus, genus Brevibacterium, genus Corynebacterium, genus Microbacterium, genus Pseudomonas and genus Synechocystis.

15. The process according to claim 14, wherein the microorganism belonging to genus Escherichia is Escherichia coli.

16. The process according to any one of claims 1 to 3, wherein the microorganism having the ability to biosynthesize mevalonic acid from acetyl-CoA is a microorganism obtainable by transfecting a DNA participating in biosynthesis of mevalonic acid from acetyl-CoA into a host microorganism using an expression vector.

17. A microorganism having the ability to biosynthesize mevalonic acid from acetyl-CoA and being obtainable by transfecting a DNA participating in biosynthesis of mevalonic acid from acetyl-CoA into a host microorganism.

18. The microorganism according to claim 17, wherein the host microorganism is a microorganism not having the ability to biosynthesize mevalonic acid from acetyl-CoA.

19. The microorganism according to claim 18, wherein the microorganism not having the ability to biosynthesize mevalonic acid from acetyl-CoA is a microorganism not having a mevalonic acid pathway.

20. The microorganism according to any one of claims 17 to 19, wherein the DNA to be transfected is one or more DNA(s) selected from the group consisting of a DNA encoding an enzyme having the activity to produce acetoacetyl-CoA from acetyl-CoA, a DNA encoding an enzyme having the activity to produce HMG-CoA from acetoacetyl-CoA, and a DNA encoding an enzyme having the activity to produce mevalonic acid from HMG-CoA.

21. The microorganism according to claim 20, wherein the DNA encoding an enzyme having the activity to produce acetoacetyl-CoA from acetyl-CoA is acetyl-CoA acetyltransferase.

22. The microorganism according to claim 20, wherein the DNA encoding an enzyme having the activity to produce HMG-CoA from acetoacetyl-CoA is HMG-CoA synthase.

23. The microorganism according to claim 20, wherein the DNA encoding an enzyme having the activity to produce mevalonic acid from HMG-CoA is HMG-CoA reductase.

24. The microorganism according to claim 20, wherein the DNA encoding an enzyme having the activity to produce acetoacetyl-CoA from acetyl-CoA and the DNA encoding an enzyme having the activity to produce mevalonic acid from HMG-CoA are a DNA encoding acetyl-CoA acetyltransferase/HMG-CoA reductase.

25. The microorganism according to any one of claims 17 to 19, wherein the DNA to be transfected is a DNA encoding acetyl-CoA acetyltransf erase, a DNA encoding HMG-CoA synthase and a DNA encoding HMG-CoA reductase.

26. The microorganism according to any one of claims 17 to 19, wherein the DNA to be transfected is a DNA encoding acetyl-CoA acetyltransferase/HMG-CoA reductase and HMG-CoA synthase.

27. The microorganism according to any one of claims 17 to 26, wherein the DNA is derived from one or more microorganism(s) selected from the group consisting of bacteria, mycobacteria, archaebacteria and yeast.

28. The microorganism according to any one of claims 17 to 26, wherein the DNA is derived from a microorganism belonging to a genus selected from the group consisting of genus Enterococcus, genus Streptococcus, genus Staphylococcus, genus Lactococcus, genus Lactobacillus, genus Streptomyces, genus Methanothermobacter, genus Methanococcus, genus Pyrococcus, genus Thermoplasma and genus Saccharomyces.

29. The microorganism according to any one of claims 17 to 19, wherein the DNA to be transfected is one or more DNA(s) selected from the group consisting of the following (a) to (j):
(a) mvaE of Enterococcus faecalis;
(b) mvaS of Enterococcus faecalis;
(c) thiL of Lactococcus lactis;
(d) mvaA of Lactococcus lactis;
(e) hmcC of Lactococcus lactis;
(f) a DNA encoding an amino acid sequence of SEQ ID NO: 2;
(g) a DNA encoding an amino acid sequence of SEQ ID NO: 4;
(h) a DNA encoding an amino acid sequence of SEQ ID NO: 12;
(i) a DNA encoding an amino acid sequence of SEQ ID NO: 14; and
(j) a DNA encoding an amino acid sequence of SEQ ID NO: 16.

30. The microorganism according to claim 19, wherein the microorganism not having a mevalonic acid pathway is a microorganism belonging to a genus selected from the group consisting of genus Escherichia, genus Serratia, genus Bacillus, genus Brevibacterium, genus Corynebacterium, genus Microbacterium, genus Pseudomonas and genus Synechocystis.

31. The microorganism according to claim 30, wherein the microorganism belonging to genus Escherichia is Escherichia coli.

32. The microorganism according to any one of claims 17 to 19, wherein the microorganism having the ability to biosynthesize mevalonic acid from acetyl-CoA is a microorganism obtainable by transfecting a DNA participating in biosynthesis of mevalonic acid from acetyl-CoA into a host microorganism using an expression vector.


SEQUENCE LISTING

<110> Kyowa Hakko Kogyo Co., Ltd.

<120> Process for producing mevalonic acid

<130> 1484

<150> JP 2002-135073
<151> 2002-05-10

<160> 22

<170> PatentIn version 3.1

<210> 1
<211> 2412
<212> DNA
<213> Enterococcus faecalis

<220>
<223> Inventor: Tabata, Kazuhiko; Hashimoto, Shinichi

<220>
<221> CDS
<222> (1)..(2412)

<400> 1
ttg aaa aca gta gtt att att gat gca tta cga aca cca att gga aaa 48
Met Lys Thr Val Val Ile Ile Asp Ala Leu Arg Thr Pro Ile Gly Lys
1               5                   10                  15

tat aaa ggc agc tta agt caa gta agt gcc gta gac tta gga aca cat 96
Tyr Lys Gly Ser Leu Ser Gln Val Ser Ala Val Asp Leu Gly Thr His
            20                  25                  30

gtt aca aca caa ctt tta aaa aga cat tcc act att tct gaa gaa att 144
Val Thr Thr Gln Leu Leu Lys Arg His Ser Thr Ile Ser Glu Glu Ile
        35                  40                  45

gat caa gta atc ttt gga aat gtt tta caa gct gga aat ggc caa aat 192
Asp Gln Val Ile Phe Gly Asn Val Leu Gln Ala Gly Asn Gly Gln Asn
    50                  55                  60

ccc gca cga caa ata gca ata aac agc ggt tta tct cat gaa att ccc 240
Pro Ala Arg Gln Ile Ala Ile Asn Ser Gly Leu Ser His Glu Ile Pro

65                  70                  75                  80

gca atg aca gtt aat gag gtc tgc gga tca gga atg aag gcc gtt att      288
Ala Met Thr Val Asn Glu Val Cys Gly Ser Gly Met Lys Ala Val Ile
                85                  90                  95

ttg gcg aaa caa ttg att caa tta gga gaa gcg gaa gtt tta att gct      336
Leu Ala Lys Gln Leu Ile Gln Leu Gly Glu Ala Glu Val Leu Ile Ala
            100                 105                 110

ggc ggg att gag aat atg tcc caa gca cct aaa tta caa cga ttt aat      384
Gly Gly Ile Glu Asn Met Ser Gln Ala Pro Lys Leu Gln Arg Phe Asn
        115                 120                 125

tac gaa aca gaa agc tat gat gcg cct ttt tct agt atg atg tac gat      432
Tyr Glu Thr Glu Ser Tyr Asp Ala Pro Phe Ser Ser Met Met Tyr Asp
    130                 135                 140

ggg tta acg gat gcc ttt agt ggt caa gca atg ggc tta act gct gaa      480
Gly Leu Thr Asp Ala Phe Ser Gly Gln Ala Met Gly Leu Thr Ala Glu
145                 150                 155                 160

aat gtg gcc gaa aag tat cat gta act aga gaa gag caa gat caa ttt      528
Asn Val Ala Glu Lys Tyr His Val Thr Arg Glu Glu Gln Asp Gln Phe
                165                 170                 175

tct gta cat tca caa tta aaa gca gct caa gca caa gca gaa ggg ata      576
Ser Val His Ser Gln Leu Lys Ala Ala Gln Ala Gln Ala Glu Gly Ile
            180                 185                 190

ttc gct gac gaa ata gcc cca tta gaa gta tca gga acg ctt gtg gag      624
Phe Ala Asp Glu Ile Ala Pro Leu Glu Val Ser Gly Thr Leu Val Glu
        195                 200                 205

aaa gat gaa ggg att cgc cct aat tcg agc gtt gag aag cta gga acg      672
Lys Asp Glu Gly Ile Arg Pro Asn Ser Ser Val Glu Lys Leu Gly Thr
    210                 215                 220

ctt aaa aca gtt ttt aaa gaa gac ggt act gta aca gca ggg aat gca      720
Leu Lys Thr Val Phe Lys Glu Asp Gly Thr Val Thr Ala Gly Asn Ala
225                 230                 235                 240

tca acc att aat gat ggg gct tct gct ttg att att gct tca caa gaa      768
Ser Thr Ile Asn Asp Gly Ala Ser Ala Leu Ile Ile Ala Ser Gln Glu
                245                 250                 255

tat gcc gaa gca cac ggt ctt cct tat tta gct att att cga gac agt 816
Tyr Ala Glu Ala His Gly Leu Pro Tyr Leu Ala Ile Ile Arg Asp Ser
            260                 265                 270

gtg gaa gtc ggt att gat cca gcc tat atg gga att tcg ccg att aaa 864
Val Glu Val Gly Ile Asp Pro Ala Tyr Met Gly Ile Ser Pro Ile Lys
        275                 280                 285

gcc att caa aaa ctg tta gcg cgc aat caa ctt act acg gaa gaa att 912
Ala Ile Gln Lys Leu Leu Ala Arg Asn Gln Leu Thr Thr Glu Glu Ile
    290                 295                 300

gat ctg tat gaa atc aac gaa gca ttt gca gca act tca atc gtg gtc 960
Asp Leu Tyr Glu Ile Asn Glu Ala Phe Ala Ala Thr Ser Ile Val Val
305                 310                 315                 320

caa aga gaa ctg gct tta cca gag gaa aag gtc aac att tat ggt ggc 1008
Gln Arg Glu Leu Ala Leu Pro Glu Glu Lys Val Asn Ile Tyr Gly Gly
                325                 330                 335

ggt att tca tta ggt cat gcg att ggt gcc aca ggt gct cgt tta tta 1056
Gly Ile Ser Leu Gly His Ala Ile Gly Ala Thr Gly Ala Arg Leu Leu
            340                 345                 350

acg agt tta agt tat caa tta aat caa aaa gaa aag aaa tat gga gtg 1104
Thr Ser Leu Ser Tyr Gln Leu Asn Gln Lys Glu Lys Lys Tyr Gly Val
        355                 360                 365

gct tct tta tgt atc ggc ggt ggc tta gga ctc gct atg cta cta gag 1152
Ala Ser Leu Cys Ile Gly Gly Gly Leu Gly Leu Ala Met Leu Leu Glu
    370                 375                 380

aga cct cag caa aaa aaa aac agc cga ttt tat caa atg agt cct gag 1200
Arg Pro Gln Gln Lys Lys Asn Ser Arg Phe Tyr Gln Met Ser Pro Glu
385                 390                 395                 400

gaa cgc ctg gct tct ctt ctt aat gaa ggc cag att tct gct gat aca 1248
Glu Arg Leu Ala Ser Leu Leu Asn Glu Gly Gln Ile Ser Ala Asp Thr
                405                 410                 415

aaa aaa gaa ttt gaa aat acg gct tta tct tcg cag att gcc aat cat 1296
Lys Lys Glu Phe Glu Asn Thr Ala Leu Ser Ser Gln Ile Ala Asn His
            420                 425                 430

atg att gaa aat caa atc agt gaa aca gaa gtg ccg atg ggc gtt ggc 1344
Met Ile Glu Asn Gln Ile Ser Glu Thr Glu Val Pro Met Gly Val Gly

435                 440                 445

tta cat tta aca gtg gac gaa act gat tat ttg gta cca atg gcg aca     1392
Leu His Leu Thr Val Asp Glu Thr Asp Tyr Leu Val Pro Met Ala Thr
    450                 455                 460

gaa gag ccc tca gtg att gcg gct ttg agt aat ggt gca aaa ata gca     1440
Glu Glu Pro Ser Val Ile Ala Ala Leu Ser Asn Gly Ala Lys Ile Ala
465                 470                 475                 480

caa gga ttt aaa aca gtg aat caa caa cgt tta atg cgt gga caa atc     1488
Gln Gly Phe Lys Thr Val Asn Gln Gln Arg Leu Met Arg Gly Gln Ile
                485                 490                 495

gtt ttt tac gat gtt gca gac gcc gag tca ttg att gat gaa cta caa     1536
Val Phe Tyr Asp Val Ala Asp Ala Glu Ser Leu Ile Asp Glu Leu Gln
            500                 505                 510

gta aga gaa acg gaa att ttt caa caa gca gag tta agt tat cca tct     1584
Val Arg Glu Thr Glu Ile Phe Gln Gln Ala Glu Leu Ser Tyr Pro Ser
        515                 520                 525

atc gtt aaa cgc ggc ggc ggc tta aga gat ttg caa tat cgt gct ttt     1632
Ile Val Lys Arg Gly Gly Gly Leu Arg Asp Leu Gln Tyr Arg Ala Phe
    530                 535                 540

gat gaa tca ttt gta tct gtc gac ttt tta gta gat gtt aag gat gca     1680
Asp Glu Ser Phe Val Ser Val Asp Phe Leu Val Asp Val Lys Asp Ala
545                 550                 555                 560

atg ggg gca aat atc gtt aac gct atg ttg gaa ggt gtg gcc gag ttg     1728
Met Gly Ala Asn Ile Val Asn Ala Met Leu Glu Gly Val Ala Glu Leu
                565                 570                 575

ttc cgt gaa tgg ttt gcg gag caa aag att tta ttc agt att tta agt     1776
Phe Arg Glu Trp Phe Ala Glu Gln Lys Ile Leu Phe Ser Ile Leu Ser
            580                 585                 590

aat tat gcc acg gag tcg gtt gtt acg atg aaa acg gct att cca gtt     1824
Asn Tyr Ala Thr Glu Ser Val Val Thr Met Lys Thr Ala Ile Pro Val
        595                 600                 605

tca cgt tta agt aag ggg agc aat ggc cgg gaa att gct gaa aaa att     1872
Ser Arg Leu Ser Lys Gly Ser Asn Gly Arg Glu Ile Ala Glu Lys Ile
    610                 615                 620

gtt tta gct tca cgc tat gct tca tta gat cct tat cgg gca gtc acg 1920
Val Leu Ala Ser Arg Tyr Ala Ser Leu Asp Pro Tyr Arg Ala Val Thr
625                 630                 635                 640

cat aac aaa ggg atc atg aat ggc att gaa gct gtc gtt tta gct aca 1968
His Asn Lys Gly Ile Met Asn Gly Ile Glu Ala Val Val Leu Ala Thr
                645                 650                 655

gga aat gat aca cgc gct gtt agc gct tct tgt cat gct ttt gcg gtg 2016
Gly Asn Asp Thr Arg Ala Val Ser Ala Ser Cys His Ala Phe Ala Val
            660                 665                 670

aag gaa ggt cgc tac caa ggt ttg act agt tgg acg ctg gat ggc gaa 2064
Lys Glu Gly Arg Tyr Gln Gly Leu Thr Ser Trp Thr Leu Asp Gly Glu
        675                 680                 685

caa cta att ggt gaa att tca gtt ccg ctt gcg tta gcc acg gtt ggc 2112
Gln Leu Ile Gly Glu Ile Ser Val Pro Leu Ala Leu Ala Thr Val Gly
    690                 695                 700

ggt gcc aca aaa gtc tta cct aaa tct caa gca gct gct gat ttg tta 2160
Gly Ala Thr Lys Val Leu Pro Lys Ser Gln Ala Ala Ala Asp Leu Leu
705                 710                 715                 720

gca gtg acg gat gca aaa gaa cta agt cga gta gta gcg gct gtt ggt 2208
Ala Val Thr Asp Ala Lys Glu Leu Ser Arg Val Val Ala Ala Val Gly
                725                 730                 735

ttg gca caa aat tta gcg gcg tta cgg gcc tta gtc tct gaa gga att 2256
Leu Ala Gln Asn Leu Ala Ala Leu Arg Ala Leu Val Ser Glu Gly Ile
            740                 745                 750

caa aaa gga cac atg gct cta caa gca cgt tct tta gcg atg acg gtc 2304
Gln Lys Gly His Met Ala Leu Gln Ala Arg Ser Leu Ala Met Thr Val
        755                 760                 765

gga gct act ggt aaa gaa gtt gag gca gtc gct caa caa tta aaa cgt 2352
Gly Ala Thr Gly Lys Glu Val Glu Ala Val Ala Gln Gln Leu Lys Arg
    770                 775                 780

caa aaa acg atg aac caa gac cga gcc ttg gct att tta aat gat tta 2400
Gln Lys Thr Met Asn Gln Asp Arg Ala Leu Ala Ile Leu Asn Asp Leu
785                 790                 795                 800

aga aaa caa taa 2412
Arg Lys Gln

<210>  2
<211>  803
<212>  PRT
<213>  Enterococcus faecalis

<400>  2

Met Lys Thr Val Val Ile Ile Asp Ala Leu Arg Thr Pro Ile Gly Lys
1               5                   10                  15

Tyr Lys Gly Ser Leu Ser Gln Val Ser Ala Val Asp Leu Gly Thr His
            20                  25                  30

Val Thr Thr Gln Leu Leu Lys Arg His Ser Thr Ile Ser Glu Glu Ile
        35                  40                  45

Asp Gln Val Ile Phe Gly Asn Val Leu Gln Ala Gly Asn Gly Gln Asn
    50                  55                  60

Pro Ala Arg Gln Ile Ala Ile Asn Ser Gly Leu Ser His Glu Ile Pro
65                  70                  75                  80

Ala Met Thr Val Asn Glu Val Cys Gly Ser Gly Met Lys Ala Val Ile
                85                  90                  95

Leu Ala Lys Gln Leu Ile Gln Leu Gly Glu Ala Glu Val Leu Ile Ala
            100                 105                 110

Gly Gly Ile Glu Asn Met Ser Gln Ala Pro Lys Leu Gln Arg Phe Asn
        115                 120                 125

Tyr Glu Thr Glu Ser Tyr Asp Ala Pro Phe Ser Ser Met Met Tyr Asp
    130                 135                 140

Gly Leu Thr Asp Ala Phe Ser Gly Gln Ala Met Gly Leu Thr Ala Glu
145                 150                 155                 160

Asn Val Ala Glu Lys Tyr His Val Thr Arg Glu Glu Gln Asp Gln Phe
                165                 170                 175

Ser Val His Ser Gln Leu Lys Ala Ala Gln Ala Gln Ala Glu Gly Ile
            180                 185                 190

Phe Ala Asp Glu Ile Ala Pro Leu Glu Val Ser Gly Thr Leu Val Glu
        195                 200                 205

Lys Asp Glu Gly Ile Arg Pro Asn Ser Ser Val Glu Lys Leu Gly Thr
    210                 215                 220

Leu Lys Thr Val Phe Lys Glu Asp Gly Thr Val Thr Ala Gly Asn Ala
225                 230                 235                 240

Ser Thr Ile Asn Asp Gly Ala Ser Ala Leu Ile Ile Ala Ser Gln Glu
                245                 250                 255

Tyr Ala Glu Ala His Gly Leu Pro Tyr Leu Ala Ile Ile Arg Asp Ser
            260                 265                 270

Val Glu Val Gly Ile Asp Pro Ala Tyr Met Gly Ile Ser Pro Ile Lys
        275                 280                 285

Ala Ile Gln Lys Leu Leu Ala Arg Asn Gln Leu Thr Thr Glu Glu Ile
    290                 295                 300

Asp Leu Tyr Glu Ile Asn Glu Ala Phe Ala Ala Thr Ser Ile Val Val
305                 310                 315                 320

Gln Arg Glu Leu Ala Leu Pro Glu Glu Lys Val Asn Ile Tyr Gly Gly
                325                 330                 335

Gly Ile Ser Leu Gly His Ala Ile Gly Ala Thr Gly Ala Arg Leu Leu
            340                 345                 350

Thr Ser Leu Ser Tyr Gln Leu Asn Gln Lys Glu Lys Lys Tyr Gly Val
        355                 360                 365

Ala Ser Leu Cys Ile Gly Gly Gly Leu Gly Leu Ala Met Leu Leu Glu
    370                 375                 380

Arg Pro Gln Gln Lys Lys Asn Ser Arg Phe Tyr Gln Met Ser Pro Glu
385                 390                 395                 400

Glu Arg Leu Ala Ser Leu Leu Asn Glu Gly Gln Ile Ser Ala Asp Thr
                405                 410                 415

Lys Lys Glu Phe Glu Asn Thr Ala Leu Ser Ser Gln Ile Ala Asn His
            420                 425                 430

Met Ile Glu Asn Gln Ile Ser Glu Thr Glu Val Pro Met Gly Val Gly
        435                 440                 445

Leu His Leu Thr Val Asp Glu Thr Asp Tyr Leu Val Pro Met Ala Thr
    450                 455                 460

Glu Glu Pro Ser Val Ile Ala Ala Leu Ser Asn Gly Ala Lys Ile Ala
465                 470                 475                 480

Gln Gly Phe Lys Thr Val Asn Gln Gln Arg Leu Met Arg Gly Gln Ile
                485                 490                 495

Val Phe Tyr Asp Val Ala Asp Ala Glu Ser Leu Ile Asp Glu Leu Gln
            500                 505                 510

Val Arg Glu Thr Glu Ile Phe Gln Gln Ala Glu Leu Ser Tyr Pro Ser
        515                 520                 525

Ile Val Lys Arg Gly Gly Gly Leu Arg Asp Leu Gln Tyr Arg Ala Phe
    530                 535                 540

Asp Glu Ser Phe Val Ser Val Asp Phe Leu Val Asp Val Lys Asp Ala
545                 550                 555                 560

Met Gly Ala Asn Ile Val Asn Ala Met Leu Glu Gly Val Ala Glu Leu
                565                 570                 575

Phe Arg Glu Trp Phe Ala Glu Gln Lys Ile Leu Phe Ser Ile Leu Ser
            580                 585                 590

Asn Tyr Ala Thr Glu Ser Val Val Thr Met Lys Thr Ala Ile Pro Val
        595                 600                 605

Ser Arg Leu Ser Lys Gly Ser Asn Gly Arg Glu Ile Ala Glu Lys Ile
    610                 615                 620

Val Leu Ala Ser Arg Tyr Ala Ser Leu Asp Pro Tyr Arg Ala Val Thr
625                 630                 635                 640

His Asn Lys Gly Ile Met Asn Gly Ile Glu Ala Val Val Leu Ala Thr
                645                 650                 655

Gly Asn Asp Thr Arg Ala Val Ser Ala Ser Cys His Ala Phe Ala Val
            660                 665                 670

Lys Glu Gly Arg Tyr Gln Gly Leu Thr Ser Trp Thr Leu Asp Gly Glu
        675                 680                 685

Gln Leu Ile Gly Glu Ile Ser Val Pro Leu Ala Leu Ala Thr Val Gly
    690                 695                 700

Gly Ala Thr Lys Val Leu Pro Lys Ser Gln Ala Ala Ala Asp Leu Leu
705                 710                 715                 720

Ala Val Thr Asp Ala Lys Glu Leu Ser Arg Val Val Ala Ala Val Gly
                725                 730                 735

Leu Ala Gln Asn Leu Ala Ala Leu Arg Ala Leu Val Ser Glu Gly Ile
            740                 745                 750

Gln Lys Gly His Met Ala Leu Gln Ala Arg Ser Leu Ala Met Thr Val
        755                 760                 765

Gly Ala Thr Gly Lys Glu Val Glu Ala Val Ala Gln Gln Leu Lys Arg
    770                 775                 780

Gln Lys Thr Met Asn Gln Asp Arg Ala Leu Ala Ile Leu Asn Asp Leu
785                 790                 795                 800

Arg Lys Gln

<210>  3
<211>  1152
<212>  DNA
<213>  Enterococcus faecalis

<220>
<221>  CDS
<222>  (1)..(1152)

<400>  3
atg aca att ggg att gat aaa att agt ttt ttt gtg ccc cct tat tat        48
Met Thr Ile Gly Ile Asp Lys Ile Ser Phe Phe Val Pro Pro Tyr Tyr
1               5                   10                  15

att gat atg acg gca ctg gct gaa gcc aga aat gta gac cct gga aaa        96
Ile Asp Met Thr Ala Leu Ala Glu Ala Arg Asn Val Asp Pro Gly Lys

            20                  25                  30

ttt cat att ggt att ggg caa gac caa atg gcg gtg aac cca atc agc 144
Phe His Ile Gly Ile Gly Gln Asp Gln Met Ala Val Asn Pro Ile Ser
        35                  40                  45

caa gat att gtg aca ttt gca gcc aat gcc gca gaa gcg atc ttg acc 192
Gln Asp Ile Val Thr Phe Ala Ala Asn Ala Ala Glu Ala Ile Leu Thr
    50                  55                  60

aaa gaa gat aaa gag gcc att gat atg gtg att gtc ggg act gag tcc 240
Lys Glu Asp Lys Glu Ala Ile Asp Met Val Ile Val Gly Thr Glu Ser
65                  70                  75                  80

agt atc gat gag tca aaa gcg gcc gca gtt gtc tta cat cgt tta atg 288
Ser Ile Asp Glu Ser Lys Ala Ala Ala Val Val Leu His Arg Leu Met
                85                  90                  95

ggg att caa cct ttc gct cgc tct ttc gaa atc aag gaa gct tgt tac 336
Gly Ile Gln Pro Phe Ala Arg Ser Phe Glu Ile Lys Glu Ala Cys Tyr
            100                 105                 110

gga gca aca gca ggc tta cag tta gct aag aat cac gta gcc tta cat 384
Gly Ala Thr Ala Gly Leu Gln Leu Ala Lys Asn His Val Ala Leu His
        115                 120                 125

cca gat aaa aaa gtc ttg gtt gta gca gca gat att gca aaa tat gga 432
Pro Asp Lys Lys Val Leu Val Val Ala Ala Asp Ile Ala Lys Tyr Gly
    130                 135                 140

tta aat tct ggc ggt gag cct aca caa gga gct ggg gcg gtt gca atg 480
Leu Asn Ser Gly Gly Glu Pro Thr Gln Gly Ala Gly Ala Val Ala Met
145                 150                 155                 160

tta gtt gct agt gaa ccg cgc atc ttg gct tta aaa gag gat aat gtg 528
Leu Val Ala Ser Glu Pro Arg Ile Leu Ala Leu Lys Glu Asp Asn Val
                165                 170                 175

atg ctg acg caa gat atc tat gac ttt tgg cgt cca aca ggc cat ccg 576
Met Leu Thr Gln Asp Ile Tyr Asp Phe Trp Arg Pro Thr Gly His Pro
            180                 185                 190

tat cct atg gtc gat ggt cct ttg tca aac gaa acc tac atc caa tct 624
Tyr Pro Met Val Asp Gly Pro Leu Ser Asn Glu Thr Tyr Ile Gln Ser
        195                 200                 205

ttt gcc caa gtc tgg gat gaa cat aaa aaa aga acc ggt ctt gat ttt 672
Phe Ala Gln Val Trp Asp Glu His Lys Lys Arg Thr Gly Leu Asp Phe
    210                 215                 220

gca gat tat gat gct tta gcg ttc cat att cct tac aca aaa atg ggc 720
Ala Asp Tyr Asp Ala Leu Ala Phe His Ile Pro Tyr Thr Lys Met Gly
225                 230                 235                 240

aaa aaa gcc tta tta gca aaa atc tcc gac caa act gaa gca gaa cag 768
Lys Lys Ala Leu Leu Ala Lys Ile Ser Asp Gln Thr Glu Ala Glu Gln
                245                 250                 255

gaa cga att tta gcc cgt tat gaa gaa agc atc atc tat agt cgt cgc 816
Glu Arg Ile Leu Ala Arg Tyr Glu Glu Ser Ile Ile Tyr Ser Arg Arg
            260                 265                 270

gta gga aac ttg tat acg ggt tca ctt tat ctg gga ctc att tcc ctt 864
Val Gly Asn Leu Tyr Thr Gly Ser Leu Tyr Leu Gly Leu Ile Ser Leu
        275                 280                 285

tta gaa aat gca acg act tta acc gca ggc aat caa att ggg tta ttc 912
Leu Glu Asn Ala Thr Thr Leu Thr Ala Gly Asn Gln Ile Gly Leu Phe
    290                 295                 300

agt tat ggt tct ggt gct gtc gct gaa ttt ttc act ggt gaa tta gta 960
Ser Tyr Gly Ser Gly Ala Val Ala Glu Phe Phe Thr Gly Glu Leu Val
305                 310                 315                 320

gct ggt tat caa aat cat tta caa aaa gaa act cat tta gca ctg cta 1008
Ala Gly Tyr Gln Asn His Leu Gln Lys Glu Thr His Leu Ala Leu Leu
                325                 330                 335

gat aat cgg aca gaa ctt tct atc gct gaa tat gaa gcc atg ttt gca 1056
Asp Asn Arg Thr Glu Leu Ser Ile Ala Glu Tyr Glu Ala Met Phe Ala
            340                 345                 350

gaa act tta gac aca gat att gat caa acg tta gaa gat gaa tta aaa 1104
Glu Thr Leu Asp Thr Asp Ile Asp Gln Thr Leu Glu Asp Glu Leu Lys
        355                 360                 365

tat agt att tct gct att aat aat acc gtt cgc tct tat cga aac taa 1152
Tyr Ser Ile Ser Ala Ile Asn Asn Thr Val Arg Ser Tyr Arg Asn
    370                 375                 380

<210> 4

<211>  383
<212>  PRT
<213>  Enterococcus faecalis

<400>  4

Met Thr Ile Gly Ile Asp Lys Ile Ser Phe Phe Val Pro Pro Tyr Tyr
1               5                   10                  15

Ile Asp Met Thr Ala Leu Ala Glu Ala Arg Asn Val Asp Pro Gly Lys
            20                  25                  30

Phe His Ile Gly Ile Gly Gln Asp Gln Met Ala Val Asn Pro Ile Ser
        35                  40                  45

Gln Asp Ile Val Thr Phe Ala Ala Asn Ala Ala Glu Ala Ile Leu Thr
    50                  55                  60

Lys Glu Asp Lys Glu Ala Ile Asp Met Val Ile Val Gly Thr Glu Ser
65                  70                  75                  80

Ser Ile Asp Glu Ser Lys Ala Ala Ala Val Val Leu His Arg Leu Met
                85                  90                  95

Gly Ile Gln Pro Phe Ala Arg Ser Phe Glu Ile Lys Glu Ala Cys Tyr
            100                 105                 110

Gly Ala Thr Ala Gly Leu Gln Leu Ala Lys Asn His Val Ala Leu His
        115                 120                 125

Pro Asp Lys Lys Val Leu Val Val Ala Ala Asp Ile Ala Lys Tyr Gly
    130                 135                 140

Leu Asn Ser Gly Gly Glu Pro Thr Gln Gly Ala Gly Ala Val Ala Met
145                 150                 155                 160

Leu Val Ala Ser Glu Pro Arg Ile Leu Ala Leu Lys Glu Asp Asn Val
                165                 170                 175

Met Leu Thr Gln Asp Ile Tyr Asp Phe Trp Arg Pro Thr Gly His Pro
            180                 185                 190

Tyr Pro Met Val Asp Gly Pro Leu Ser Asn Glu Thr Tyr Ile Gln Ser
        195                 200                 205

Phe Ala Gln Val Trp Asp Glu His Lys Lys Arg Thr Gly Leu Asp Phe
    210                 215                 220

Ala Asp Tyr Asp Ala Leu Ala Phe His Ile Pro Tyr Thr Lys Met Gly
225                 230                 235                 240

Lys Lys Ala Leu Leu Ala Lys Ile Ser Asp Gln Thr Glu Ala Glu Gln
                245                 250                 255

Glu Arg Ile Leu Ala Arg Tyr Glu Glu Ser Ile Ile Tyr Ser Arg Arg
            260                 265                 270

Val Gly Asn Leu Tyr Thr Gly Ser Leu Tyr Leu Gly Leu Ile Ser Leu
        275                 280                 285

Leu Glu Asn Ala Thr Thr Leu Thr Ala Gly Asn Gln Ile Gly Leu Phe
    290                 295                 300

Ser Tyr Gly Ser Gly Ala Val Ala Glu Phe Phe Thr Gly Glu Leu Val
305                 310                 315                 320

Ala Gly Tyr Gln Asn His Leu Gln Lys Glu Thr His Leu Ala Leu Leu
                325                 330                 335

Asp Asn Arg Thr Glu Leu Ser Ile Ala Glu Tyr Glu Ala Met Phe Ala
            340                 345                 350

Glu Thr Leu Asp Thr Asp Ile Asp Gln Thr Leu Glu Asp Glu Leu Lys
        355                 360                 365

Tyr Ser Ile Ser Ala Ile Asn Asn Thr Val Arg Ser Tyr Arg Asn
    370                 375                 380

<210>  5
<211>  33
<212>  DNA
<213>  Artificial

<220>
<223>  primer A

<400>  5
tatatcgata tttagatgaa aacagtagtt att                                    33

<210>  6
<211>  33
<212>  DNA
<213>  Artificial

<220>
<223>  primer B

<400>  6
aatggatcct tattgttttc ttaaatcatt taa                                    33

<210>  7
<211>  33
<212>  DNA
<213>  Artificial

<220>
<223>  primer C

<400>  7
tatagatcta aggaaggagt taaagaaatg aca                                    33

<210> 8
<211> 32
<212> DNA
<213> Artificial

<220>
<223> primer D

<400> 8
tttggatcct tagtttcgat aagagcgaac gg 32


<210> 9
<211> 2418
<212> DNA
<213> Enterococcus faecalis

<220>
<221> CDS
<222> (7)..(2418)

<400> 9
atttag ttg aaa aca gta gtt att att gat gca tta cga aca cca att 48
Met Lys Thr Val Val Ile Ile Asp Ala Leu Arg Thr Pro Ile
1               5                   10

gga aaa tat aaa ggc agc tta agt caa gta agt gcc gta gac tta gga 96
Gly Lys Tyr Lys Gly Ser Leu Ser Gln Val Ser Ala Val Asp Leu Gly
15                  20                  25                  30

aca cat gtt aca aca caa ctt tta aaa aga cat tcc act att tct gaa 144
Thr His Val Thr Thr Gln Leu Leu Lys Arg His Ser Thr Ile Ser Glu
                35                  40                  45

gaa att gat caa gta atc ttt gga aat gtt tta caa gct gga aat ggc 192
Glu Ile Asp Gln Val Ile Phe Gly Asn Val Leu Gln Ala Gly Asn Gly
            50                  55                  60

caa aat ccc gca cga caa ata gca ata aac agc ggt tta tct cat gaa 240
Gln Asn Pro Ala Arg Gln Ile Ala Ile Asn Ser Gly Leu Ser His Glu
        65                  70                  75

att ccc gca atg aca gtt aat gag gtc tgc gga tca gga atg aag gcc 288
Ile Pro Ala Met Thr Val Asn Glu Val Cys Gly Ser Gly Met Lys Ala
    80                  85                  90

gtt att ttg gcg aaa caa ttg att caa tta gga gaa gcg gaa gtt tta 336
Val Ile Leu Ala Lys Gln Leu Ile Gln Leu Gly Glu Ala Glu Val Leu
95                  100                 105                 110

att gct ggc ggg att gag aat atg tcc caa gca cct aaa tta caa cga 384
Ile Ala Gly Gly Ile Glu Asn Met Ser Gln Ala Pro Lys Leu Gln Arg
                115                 120                 125

ttt aat tac gaa aca gaa agc tat gat gcg cct ttt tct agt atg atg 432
Phe Asn Tyr Glu Thr Glu Ser Tyr Asp Ala Pro Phe Ser Ser Met Met
            130                 135                 140

tac gat ggg tta acg gat gcc ttt agt ggt caa gca atg ggc tta act 480
Tyr Asp Gly Leu Thr Asp Ala Phe Ser Gly Gln Ala Met Gly Leu Thr
        145                 150                 155

gct gaa aat gtg gcc gaa aag tat cat gta act aga gaa gag caa gat 528
Ala Glu Asn Val Ala Glu Lys Tyr His Val Thr Arg Glu Glu Gln Asp
    160                 165                 170

caa ttt tct gta cat tca caa tta aaa gca gct caa gca caa gca gaa 576
Gln Phe Ser Val His Ser Gln Leu Lys Ala Ala Gln Ala Gln Ala Glu
175                 180                 185                 190

ggg ata ttc gct gac gaa ata gcc cca tta gaa gta tca gga acg ctt 624
Gly Ile Phe Ala Asp Glu Ile Ala Pro Leu Glu Val Ser Gly Thr Leu
                195                 200                 205

gtg gag aaa gat gaa ggg att cgc cct aat tcg agc gtt gag aag cta 672
Val Glu Lys Asp Glu Gly Ile Arg Pro Asn Ser Ser Val Glu Lys Leu
            210                 215                 220

gga acg ctt aaa aca gtt ttt aaa gaa gac ggt act gta aca gca ggg 720
Gly Thr Leu Lys Thr Val Phe Lys Glu Asp Gly Thr Val Thr Ala Gly
        225                 230                 235

aat gca tca acc att aat gat ggg gct tct gct ttg att att gct tca 768
Asn Ala Ser Thr Ile Asn Asp Gly Ala Ser Ala Leu Ile Ile Ala Ser
    240                 245                 250

caa gaa tat gcc gaa gca cac ggt ctt cct tat tta gct att att cga 816
Gln Glu Tyr Ala Glu Ala His Gly Leu Pro Tyr Leu Ala Ile Ile Arg
255                 260                 265                 270

gac agt gtg gaa gtc ggt att gat cca gcc tat atg gga att tcg ccg 864
Asp Ser Val Glu Val Gly Ile Asp Pro Ala Tyr Met Gly Ile Ser Pro

                275                 280                 285

att aaa gcc att caa aaa ctg tta gcg cgc aat caa ctt act acg gaa 912
Ile Lys Ala Ile Gln Lys Leu Leu Ala Arg Asn Gln Leu Thr Thr Glu
            290                 295                 300

gaa att gat ctg tat gaa atc aac gaa gca ttt gca gca act tca atc 960
Glu Ile Asp Leu Tyr Glu Ile Asn Glu Ala Phe Ala Ala Thr Ser Ile
        305                 310                 315

gtg gtc caa aga gaa ctg gct tta cca gag gaa aag gtc aac att tat 1008
Val Val Gln Arg Glu Leu Ala Leu Pro Glu Glu Lys Val Asn Ile Tyr
    320                 325                 330

ggt ggc ggt att tca tta ggt cat gcg att ggt gcc aca ggt gct cgt 1056
Gly Gly Gly Ile Ser Leu Gly His Ala Ile Gly Ala Thr Gly Ala Arg
335                 340                 345                 350

tta tta acg agt tta agt tat caa tta aat caa aaa gaa aag aaa tat 1104
Leu Leu Thr Ser Leu Ser Tyr Gln Leu Asn Gln Lys Glu Lys Lys Tyr
                355                 360                 365

gga gtg gct tct tta tgt atc ggc ggt ggc tta gga ctc gct atg cta 1152
Gly Val Ala Ser Leu Cys Ile Gly Gly Gly Leu Gly Leu Ala Met Leu
            370                 375                 380

cta gag aga cct cag caa aaa aaa aac agc cga ttt tat caa atg agt 1200
Leu Glu Arg Pro Gln Gln Lys Lys Asn Ser Arg Phe Tyr Gln Met Ser
        385                 390                 395

cct gag gaa cgc ctg gct tct ctt ctt aat gaa ggc cag att tct gct 1248
Pro Glu Glu Arg Leu Ala Ser Leu Leu Asn Glu Gly Gln Ile Ser Ala
    400                 405                 410

gat aca aaa aaa gaa ttt gaa aat acg gct tta tct tcg cag att gcc 1296
Asp Thr Lys Lys Glu Phe Glu Asn Thr Ala Leu Ser Ser Gln Ile Ala
415                 420                 425                 430

aat cat atg att gaa aat caa atc agt gaa aca gaa gtg ccg atg ggc 1344
Asn His Met Ile Glu Asn Gln Ile Ser Glu Thr Glu Val Pro Met Gly
                435                 440                 445

gtt ggc tta cat tta aca gtg gac gaa act gat tat ttg gta cca atg 1392
Val Gly Leu His Leu Thr Val Asp Glu Thr Asp Tyr Leu Val Pro Met
            450                 455                 460

gcg aca gaa gag ccc tca gtg att gcg gct ttg agt aat ggt gca aaa     1440
Ala Thr Glu Glu Pro Ser Val Ile Ala Ala Leu Ser Asn Gly Ala Lys
        465                 470                 475

ata gca caa gga ttt aaa aca gtg aat caa caa cgt tta atg cgt gga     1488
Ile Ala Gln Gly Phe Lys Thr Val Asn Gln Gln Arg Leu Met Arg Gly
    480                 485                 490

caa atc gtt ttt tac gat gtt gca gac gcc gag tca ttg att gat gaa     1536
Gln Ile Val Phe Tyr Asp Val Ala Asp Ala Glu Ser Leu Ile Asp Glu
495                 500                 505                 510

cta caa gta aga gaa acg gaa att ttt caa caa gca gag tta agt tat     1584
Leu Gln Val Arg Glu Thr Glu Ile Phe Gln Gln Ala Glu Leu Ser Tyr
                515                 520                 525

cca tct atc gtt aaa cgc ggc ggc ggc tta aga gat ttg caa tat cgt     1632
Pro Ser Ile Val Lys Arg Gly Gly Gly Leu Arg Asp Leu Gln Tyr Arg
            530                 535                 540

gct ttt gat gaa tca ttt gta tct gtc gac ttt tta gta gat gtt aag     1680
Ala Phe Asp Glu Ser Phe Val Ser Val Asp Phe Leu Val Asp Val Lys
        545                 550                 555

gat gca atg ggg gca aat atc gtt aac gct atg ttg gaa ggt gtg gcc     1728
Asp Ala Met Gly Ala Asn Ile Val Asn Ala Met Leu Glu Gly Val Ala
    560                 565                 570

gag ttg ttc cgt gaa tgg ttt gcg gag caa aag att tta ttc agt att     1776
Glu Leu Phe Arg Glu Trp Phe Ala Glu Gln Lys Ile Leu Phe Ser Ile
575                 580                 585                 590

tta agt aat tat gcc acg gag tcg gtt gtt acg atg aaa acg gct att     1824
Leu Ser Asn Tyr Ala Thr Glu Ser Val Val Thr Met Lys Thr Ala Ile
                595                 600                 605

cca gtt tca cgt tta agt aag ggg agc aat ggc cgg gaa att gct gaa     1872
Pro Val Ser Arg Leu Ser Lys Gly Ser Asn Gly Arg Glu Ile Ala Glu
            610                 615                 620

aaa att gtt tta gct tca cgc tat gct tca tta gat cct tat cgg gca     1920
Lys Ile Val Leu Ala Ser Arg Tyr Ala Ser Leu Asp Pro Tyr Arg Ala
        625                 630                 635

gtc acg cat aac aaa ggg atc atg aat ggc att gaa gct gtc gtt tta     1968
Val Thr His Asn Lys Gly Ile Met Asn Gly Ile Glu Ala Val Val Leu

640                 645                 650

gct aca gga aat gat aca cgc gct gtt agc gct tct tgt cat gct ttt      2016
Ala Thr Gly Asn Asp Thr Arg Ala Val Ser Ala Ser Cys His Ala Phe
655                 660                 665                 670

gcg gtg aag gaa ggt cgc tac caa ggt ttg act agt tgg acg ctg gat      2064
Ala Val Lys Glu Gly Arg Tyr Gln Gly Leu Thr Ser Trp Thr Leu Asp
                675                 680                 685

ggc gaa caa cta att ggt gaa att tca gtt ccg ctt gcg tta gcc acg      2112
Gly Glu Gln Leu Ile Gly Glu Ile Ser Val Pro Leu Ala Leu Ala Thr
            690                 695                 700

gtt ggc ggt gcc aca aaa gtc tta cct aaa tct caa gca gct gct gat      2160
Val Gly Gly Ala Thr Lys Val Leu Pro Lys Ser Gln Ala Ala Ala Asp
        705                 710                 715

ttg tta gca gtg acg gat gca aaa gaa cta agt cga gta gta gcg gct      2208
Leu Leu Ala Val Thr Asp Ala Lys Glu Leu Ser Arg Val Val Ala Ala
    720                 725                 730

gtt ggt ttg gca caa aat tta gcg gcg tta cgg gcc tta gtc tct gaa      2256
Val Gly Leu Ala Gln Asn Leu Ala Ala Leu Arg Ala Leu Val Ser Glu
735                 740                 745                 750

gga att caa aaa gga cac atg gct cta caa gca cgt tct tta gcg atg      2304
Gly Ile Gln Lys Gly His Met Ala Leu Gln Ala Arg Ser Leu Ala Met
                755                 760                 765

acg gtc gga gct act ggt aaa gaa gtt gag gca gtc gct caa caa tta      2352
Thr Val Gly Ala Thr Gly Lys Glu Val Glu Ala Val Ala Gln Gln Leu
            770                 775                 780

aaa cgt caa aaa acg atg aac caa gac cga gcc ttg gct att tta aat      2400
Lys Arg Gln Lys Thr Met Asn Gln Asp Arg Ala Leu Ala Ile Leu Asn
        785                 790                 795

gat tta aga aaa caa taa                                              2418
Asp Leu Arg Lys Gln
    800

<210>  10
<211>  1166
<212>  DNA

<213>  Enterococcus faecalis

<220>
<221>  CDS
<222>  (15)..(1166)

<400>  10
aaggagttaa agaa atg aca att ggg att gat aaa att agt ttt ttt gtg      50
                Met Thr Ile Gly Ile Asp Lys Ile Ser Phe Phe Val
                1               5                   10

ccc cct tat tat att gat atg acg gca ctg gct gaa gcc aga aat gta      98
Pro Pro Tyr Tyr Ile Asp Met Thr Ala Leu Ala Glu Ala Arg Asn Val
        15                  20                  25

gac cct gga aaa ttt cat att ggt att ggg caa gac caa atg gcg gtg      146
Asp Pro Gly Lys Phe His Ile Gly Ile Gly Gln Asp Gln Met Ala Val
    30                  35                  40

aac cca atc agc caa gat att gtg aca ttt gca gcc aat gcc gca gaa      194
Asn Pro Ile Ser Gln Asp Ile Val Thr Phe Ala Ala Asn Ala Ala Glu
45                  50                  55                  60

gcg atc ttg acc aaa gaa gat aaa gag gcc att gat atg gtg att gtc      242
Ala Ile Leu Thr Lys Glu Asp Lys Glu Ala Ile Asp Met Val Ile Val
                65                  70                  75

ggg act gag tcc agt atc gat gag tca aaa gcg gcc gca gtt gtc tta      290
Gly Thr Glu Ser Ser Ile Asp Glu Ser Lys Ala Ala Ala Val Val Leu
            80                  85                  90

cat cgt tta atg ggg att caa cct ttc gct cgc tct ttc gaa atc aag      338
His Arg Leu Met Gly Ile Gln Pro Phe Ala Arg Ser Phe Glu Ile Lys
        95                  100                 105

gaa gct tgt tac gga gca aca gca ggc tta cag tta gct aag aat cac      386
Glu Ala Cys Tyr Gly Ala Thr Ala Gly Leu Gln Leu Ala Lys Asn His
    110                 115                 120

gta gcc tta cat cca gat aaa aaa gtc ttg gtt gta gca gca gat att      434
Val Ala Leu His Pro Asp Lys Lys Val Leu Val Val Ala Ala Asp Ile
125                 130                 135                 140

gca aaa tat gga tta aat tct ggc ggt gag cct aca caa gga gct ggg      482
Ala Lys Tyr Gly Leu Asn Ser Gly Gly Glu Pro Thr Gln Gly Ala Gly
                145                 150                 155

gcg gtt gca atg tta gtt gct agt gaa ccg cgc atc ttg gct tta aaa 530
Ala Val Ala Met Leu Val Ala Ser Glu Pro Arg Ile Leu Ala Leu Lys
            160                 165                 170

gag gat aat gtg atg ctg acg caa gat atc tat gac ttt tgg cgt cca 578
Glu Asp Asn Val Met Leu Thr Gln Asp Ile Tyr Asp Phe Trp Arg Pro
        175                 180                 185

aca ggc cat ccg tat cct atg gtc gat ggt cct ttg tca aac gaa acc 626
Thr Gly His Pro Tyr Pro Met Val Asp Gly Pro Leu Ser Asn Glu Thr
    190                 195                 200

tac atc caa tct ttt gcc caa gtc tgg gat gaa cat aaa aaa aga acc 674
Tyr Ile Gln Ser Phe Ala Gln Val Trp Asp Glu His Lys Lys Arg Thr
205                 210                 215                 220

ggt ctt gat ttt gca gat tat gat gct tta gcg ttc cat att cct tac 722
Gly Leu Asp Phe Ala Asp Tyr Asp Ala Leu Ala Phe His Ile Pro Tyr
                225                 230                 235

aca aaa atg ggc aaa aaa gcc tta tta gca aaa atc tcc gac caa act 770
Thr Lys Met Gly Lys Lys Ala Leu Leu Ala Lys Ile Ser Asp Gln Thr
            240                 245                 250

gaa gca gaa cag gaa cga att tta gcc cgt tat gaa gaa agc atc atc 818
Glu Ala Glu Gln Glu Arg Ile Leu Ala Arg Tyr Glu Glu Ser Ile Ile
        255                 260                 265

tat agt cgt cgc gta gga aac ttg tat acg ggt tca ctt tat ctg gga 866
Tyr Ser Arg Arg Val Gly Asn Leu Tyr Thr Gly Ser Leu Tyr Leu Gly
    270                 275                 280

ctc att tcc ctt tta gaa aat gca acg act tta acc gca ggc aat caa 914
Leu Ile Ser Leu Leu Glu Asn Ala Thr Thr Leu Thr Ala Gly Asn Gln
285                 290                 295                 300

att ggg tta ttc agt tat ggt tct ggt gct gtc gct gaa ttt ttc act 962
Ile Gly Leu Phe Ser Tyr Gly Ser Gly Ala Val Ala Glu Phe Phe Thr
                305                 310                 315

ggt gaa tta gta gct ggt tat caa aat cat tta caa aaa gaa act cat 1010
Gly Glu Leu Val Ala Gly Tyr Gln Asn His Leu Gln Lys Glu Thr His
            320                 325                 330

tta gca ctg cta gat aat cgg aca gaa ctt tct atc gct gaa tat gaa 1058

Leu Ala Leu Leu Asp Asn Arg Thr Glu Leu Ser Ile Ala Glu Tyr Glu
        335                 340                 345

gcc atg ttt gca gaa act tta gac aca gat att gat caa acg tta gaa 1106
Ala Met Phe Ala Glu Thr Leu Asp Thr Asp Ile Asp Gln Thr Leu Glu
    350                 355                 360

gat gaa tta aaa tat agt att tct gct att aat aat acc gtt cgc tct 1154
Asp Glu Leu Lys Tyr Ser Ile Ser Ala Ile Asn Asn Thr Val Arg Ser
365                 370                 375                 380

tat cga aac taa 1166
Tyr Arg Asn

<210> 11
<211> 1140
<212> DNA
<213> Lactococcus lactis

<220>
<221> CDS
<222> (1)..(1140)

<400> 11
gtg aaa gaa att gta atc att gac gcc tta aga aca cca gtt gga aga 48
Met Lys Glu Ile Val Ile Ile Asp Ala Leu Arg Thr Pro Val Gly Arg
1               5                   10                  15

tac gac ggt gct ctt gct gaa tat tca gca gca gaa ctt ggg aca cat 96
Tyr Asp Gly Ala Leu Ala Glu Tyr Ser Ala Ala Glu Leu Gly Thr His
            20                  25                  30

gtt gtt tca aac tta ttg tca aaa cat gaa aat ata aaa tca gat gtt 144
Val Val Ser Asn Leu Leu Ser Lys His Glu Asn Ile Lys Ser Asp Val
        35                  40                  45

gaa caa gtt att ttt ggg aat gtt ctt caa gct ggt aat ggt caa aat 192
Glu Gln Val Ile Phe Gly Asn Val Leu Gln Ala Gly Asn Gly Gln Asn
    50                  55                  60

ata gca cgt caa att gcg att cat agt ggt cta tct aat aca gtg ccc 240
Ile Ala Arg Gln Ile Ala Ile His Ser Gly Leu Ser Asn Thr Val Pro
65                  70                  75                  80

gct tca aca atc aac gaa gtt tgt ggt tca gga atg aaa gca gtg att 288
Ala Ser Thr Ile Asn Glu Val Cys Gly Ser Gly Met Lys Ala Val Ile
                85                  90                  95

tta gct aaa caa ctt tta cag ctt ggt gaa gca gaa gtt gta atc gct 336
Leu Ala Lys Gln Leu Leu Gln Leu Gly Glu Ala Glu Val Val Ile Ala
            100                 105                 110

ggt ggg aca gaa tct atg tct aat gcc cca att ata aag aat cga aaa 384
Gly Gly Thr Glu Ser Met Ser Asn Ala Pro Ile Ile Lys Asn Arg Lys
        115                 120                 125

act caa gaa gaa aca tta agt atg tta aat gat ggc tta gtt gat gcc 432
Thr Gln Glu Glu Thr Leu Ser Met Leu Asn Asp Gly Leu Val Asp Ala
    130                 135                 140

ttc tca gga att tct atg ggg att att ggt gaa act att gct gaa caa 480
Phe Ser Gly Ile Ser Met Gly Ile Ile Gly Glu Thr Ile Ala Glu Gln
145                 150                 155                 160

ttt gat ata agt cga gca gac caa gac gct ttt gcg caa aac tct caa 528
Phe Asp Ile Ser Arg Ala Asp Gln Asp Ala Phe Ala Gln Asn Ser Gln
                165                 170                 175

gaa aaa gcg gtc gct gca agt caa gcc gga att ttt aaa gaa gag att 576
Glu Lys Ala Val Ala Ala Ser Gln Ala Gly Ile Phe Lys Glu Glu Ile
            180                 185                 190

gtt gcg att gga gaa gtc gga gtc gac gaa aca cca cgt cct tct tct 624
Val Ala Ile Gly Glu Val Gly Val Asp Glu Thr Pro Arg Pro Ser Ser
        195                 200                 205

agt tta gaa aaa tta gcc act tta agg aca gct ttc aaa gaa aat gga 672
Ser Leu Glu Lys Leu Ala Thr Leu Arg Thr Ala Phe Lys Glu Asn Gly
    210                 215                 220

act gtg aca gca ggt aat tct tct cct gtt aat gat gga gct tca gct 720
Thr Val Thr Ala Gly Asn Ser Ser Pro Val Asn Asp Gly Ala Ser Ala
225                 230                 235                 240

gtc att ctt gca acg aaa gac tat gct gaa aaa cat ggg att ccg tac 768
Val Ile Leu Ala Thr Lys Asp Tyr Ala Glu Lys His Gly Ile Pro Tyr
                245                 250                 255

cta gca gaa cta gtt gat agt aca gaa gtt gga att gac cct gct att 816
Leu Ala Glu Leu Val Asp Ser Thr Glu Val Gly Ile Asp Pro Ala Ile

260                 265                 270

atg gga gtt tca cca att gaa gct atc aaa aaa tta att aat cgt tca        864
Met Gly Val Ser Pro Ile Glu Ala Ile Lys Lys Leu Ile Asn Arg Ser
        275                 280                 285

gag ctt gaa ctt tca gat att gac ctg ttt gaa att aat gaa gca ttt        912
Glu Leu Glu Leu Ser Asp Ile Asp Leu Phe Glu Ile Asn Glu Ala Phe
    290                 295                 300

gca gca tca agt ctt gct gtc aat cgt gaa ctt ggt tta aat gaa tca        960
Ala Ala Ser Ser Leu Ala Val Asn Arg Glu Leu Gly Leu Asn Glu Ser
305                 310                 315                 320

caa gtt aac att tat ggt ggt gca att gca tta ggt cat gct atc ggt       1008
Gln Val Asn Ile Tyr Gly Gly Ala Ile Ala Leu Gly His Ala Ile Gly
                325                 330                 335

tca agt ggt tct aaa att ctc acg act ttg tct tat gcc tta aaa cgt       1056
Ser Ser Gly Ser Lys Ile Leu Thr Thr Leu Ser Tyr Ala Leu Lys Arg
            340                 345                 350

gag caa aaa cgc tat gga att gct agt ctt tgt atc ggt gga ggc cta       1104
Glu Gln Lys Arg Tyr Gly Ile Ala Ser Leu Cys Ile Gly Gly Gly Leu
        355                 360                 365

ggt ttg gct gtt ctt tta aaa aat cct aac ctt taa                       1140
Gly Leu Ala Val Leu Leu Lys Asn Pro Asn Leu
    370                 375

<210>  12
<211>  379
<212>  PRT
<213>  Lactococcus lactis

<400>  12

Met Lys Glu Ile Val Ile Ile Asp Ala Leu Arg Thr Pro Val Gly Arg
1               5                   10                  15

Tyr Asp Gly Ala Leu Ala Glu Tyr Ser Ala Ala Glu Leu Gly Thr His
            20                  25                  30

Val Val Ser Asn Leu Leu Ser Lys His Glu Asn Ile Lys Ser Asp Val
        35                  40                  45

Glu Gln Val Ile Phe Gly Asn Val Leu Gln Ala Gly Asn Gly Gln Asn
    50                  55                  60

Ile Ala Arg Gln Ile Ala Ile His Ser Gly Leu Ser Asn Thr Val Pro
65                  70                  75                  80

Ala Ser Thr Ile Asn Glu Val Cys Gly Ser Gly Met Lys Ala Val Ile
                85                  90                  95

Leu Ala Lys Gln Leu Leu Gln Leu Gly Glu Ala Glu Val Val Ile Ala
            100                 105                 110

Gly Gly Thr Glu Ser Met Ser Asn Ala Pro Ile Ile Lys Asn Arg Lys
        115                 120                 125

Thr Gln Glu Glu Thr Leu Ser Met Leu Asn Asp Gly Leu Val Asp Ala
    130                 135                 140

Phe Ser Gly Ile Ser Met Gly Ile Ile Gly Glu Thr Ile Ala Glu Gln
145                 150                 155                 160

Phe Asp Ile Ser Arg Ala Asp Gln Asp Ala Phe Ala Gln Asn Ser Gln
                165                 170                 175

Glu Lys Ala Val Ala Ala Ser Gln Ala Gly Ile Phe Lys Glu Glu Ile
            180                 185                 190

Val Ala Ile Gly Glu Val Gly Val Asp Glu Thr Pro Arg Pro Ser Ser
        195                 200                 205

Ser Leu Glu Lys Leu Ala Thr Leu Arg Thr Ala Phe Lys Glu Asn Gly
    210                 215                 220

Thr Val Thr Ala Gly Asn Ser Ser Pro Val Asn Asp Gly Ala Ser Ala
225                 230                 235                 240

Val Ile Leu Ala Thr Lys Asp Tyr Ala Glu Lys His Gly Ile Pro Tyr
                245                 250                 255

Leu Ala Glu Leu Val Asp Ser Thr Glu Val Gly Ile Asp Pro Ala Ile
            260                 265                 270

Met Gly Val Ser Pro Ile Glu Ala Ile Lys Lys Leu Ile Asn Arg Ser
        275                 280                 285

Glu Leu Glu Leu Ser Asp Ile Asp Leu Phe Glu Ile Asn Glu Ala Phe
    290                 295                 300

Ala Ala Ser Ser Leu Ala Val Asn Arg Glu Leu Gly Leu Asn Glu Ser
305                 310                 315                 320

Gln Val Asn Ile Tyr Gly Gly Ala Ile Ala Leu Gly His Ala Ile Gly
                325                 330                 335

Ser Ser Gly Ser Lys Ile Leu Thr Thr Leu Ser Tyr Ala Leu Lys Arg
            340                 345                 350

Glu Gln Lys Arg Tyr Gly Ile Ala Ser Leu Cys Ile Gly Gly Gly Leu
        355                 360                 365

Gly Leu Ala Val Leu Leu Lys Asn Pro Asn Leu
    370                 375

<210>  13
<211>  1233
<212>  DNA
<213>  Lactococcus lactis

<220>
<221>  CDS
<222>  (1)..(1233)

<400>  13
atg aga aaa aaa ttt tat caa atg tcg ccc aaa gag cga tta gat tca      48
Met Arg Lys Lys Phe Tyr Gln Met Ser Pro Lys Glu Arg Leu Asp Ser
1               5                   10                  15

ttg aat ctg tca gaa gat act cag gaa gtt ttg agt gaa atg gct tta      96
Leu Asn Leu Ser Glu Asp Thr Gln Glu Val Leu Ser Glu Met Ala Leu
            20                  25                  30

gat aca aat att tta aac aat cta att gaa aat caa att tcc gaa ttt      144
Asp Thr Asn Ile Leu Asn Asn Leu Ile Glu Asn Gln Ile Ser Glu Phe
        35                  40                  45

gaa ttg cca atg gga ctt gct caa aat ttt gtc att aat gga aaa gaa      192
Glu Leu Pro Met Gly Leu Ala Gln Asn Phe Val Ile Asn Gly Lys Glu
    50                  55                  60

tac atc gtt ccg atg gtt aca gaa gaa cca tca gtt ata gct gct gcg      240
Tyr Ile Val Pro Met Val Thr Glu Glu Pro Ser Val Ile Ala Ala Ala
65                  70                  75                  80

agt aat gga gca aaa ata gcg gaa agt ttt acg gct aaa ata gat gaa      288
Ser Asn Gly Ala Lys Ile Ala Glu Ser Phe Thr Ala Lys Ile Asp Glu
                85                  90                  95

cgg tta atg cgt ggg caa att gta ttt tat gac gta aag aaa cca gaa      336
Arg Leu Met Arg Gly Gln Ile Val Phe Tyr Asp Val Lys Lys Pro Glu
            100                 105                 110

gaa att att aag aaa ata tct gaa tgc aaa aat gaa ata ttt gaa caa      384
Glu Ile Ile Lys Lys Ile Ser Glu Cys Lys Asn Glu Ile Phe Glu Gln
        115                 120                 125

gct aag ctt tcg tat cct tca att att aag cgt ggg ggc ggt ttg aga      432
Ala Lys Leu Ser Tyr Pro Ser Ile Ile Lys Arg Gly Gly Gly Leu Arg
    130                 135                 140

gaa att tcg agc aga ctt ttt tca agt gag aaa ttt att tca gtt gat      480
Glu Ile Ser Ser Arg Leu Phe Ser Ser Glu Lys Phe Ile Ser Val Asp
145                 150                 155                 160

ttc aaa gtg gat gtc aaa gat gca atg ggt gcc aat att att aat tct 528
Phe Lys Val Asp Val Lys Asp Ala Met Gly Ala Asn Ile Ile Asn Ser
                165                 170                 175

ata tta gaa ggt gta gca gaa ctt ttt cgt ggc tgg ttt tct gag gaa 576
Ile Leu Glu Gly Val Ala Glu Leu Phe Arg Gly Trp Phe Ser Glu Glu
            180                 185                 190

aaa att ctg ttt agc atc ctt tca aac tat gca act gaa tct tta gta 624
Lys Ile Leu Phe Ser Ile Leu Ser Asn Tyr Ala Thr Glu Ser Leu Val
        195                 200                 205

aaa gtg agc tgt gaa att tca gtt gat gct ctt tca aaa aaa aca aat 672
Lys Val Ser Cys Glu Ile Ser Val Asp Ala Leu Ser Lys Lys Thr Asn
    210                 215                 220

ggt tta gaa att gct caa aaa att gca gtt gct tca caa tac tca aaa 720
Gly Leu Glu Ile Ala Gln Lys Ile Ala Val Ala Ser Gln Tyr Ser Lys
225                 230                 235                 240

att gat cct tac cgt gct agc aca cac aat aaa ggg att atg aat ggg 768
Ile Asp Pro Tyr Arg Ala Ser Thr His Asn Lys Gly Ile Met Asn Gly
                245                 250                 255

att aat gct gta att ctt gca aca gga aat gat acg cga gct att tca 816
Ile Asn Ala Val Ile Leu Ala Thr Gly Asn Asp Thr Arg Ala Ile Ser
            260                 265                 270

gca gca att cat gct tat gca gca aaa gaa gga aca tat caa ggt ttg 864
Ala Ala Ile His Ala Tyr Ala Ala Lys Glu Gly Thr Tyr Gln Gly Leu
        275                 280                 285

gct aaa tgg gaa gtt cat gcc gaa aag tta ttt gga gag tta gaa att 912
Ala Lys Trp Glu Val His Ala Glu Lys Leu Phe Gly Glu Leu Glu Ile
    290                 295                 300

cct tta cct gtg gcg acc gtt ggt ggt ggg gtg aaa gtc tta cct aaa 960
Pro Leu Pro Val Ala Thr Val Gly Gly Gly Val Lys Val Leu Pro Lys
305                 310                 315                 320

gca caa gcg gca atg gaa att tta gga atc aca gat gct aga gaa ctg 1008
Ala Gln Ala Ala Met Glu Ile Leu Gly Ile Thr Asp Ala Arg Glu Leu
                325                 330                 335

gcc aaa gtt atc gcc gca gtt ggt tta gca caa aat tta gca gct ttg 1056
Ala Lys Val Ile Ala Ala Val Gly Leu Ala Gln Asn Leu Ala Ala Leu

340                 345                 350

cgc gca ctt gtt tca gaa gga att caa caa gga cat atg agc tta cag      1104
Arg Ala Leu Val Ser Glu Gly Ile Gln Gln Gly His Met Ser Leu Gln
        355                 360                 365

gct cgc tct ttg gca tta tcc gtg ggt gca aaa gca gat gag ata gca      1152
Ala Arg Ser Leu Ala Leu Ser Val Gly Ala Lys Ala Asp Glu Ile Ala
    370                 375                 380

gtc ata agt cag caa ctt cgg cag gaa aaa gtg atg aat caa gaa gta      1200
Val Ile Ser Gln Gln Leu Arg Gln Glu Lys Val Met Asn Gln Glu Val
385                 390                 395                 400

gct cgc cgt tta tta aat agt ctc aga aac taa                          1233
Ala Arg Arg Leu Leu Asn Ser Leu Arg Asn
                405                 410

<210>  14
<211>  410
<212>  PRT
<213>  Lactococcus lactis

<400>  14

Met Arg Lys Lys Phe Tyr Gln Met Ser Pro Lys Glu Arg Leu Asp Ser
1               5                   10                  15

Leu Asn Leu Ser Glu Asp Thr Gln Glu Val Leu Ser Glu Met Ala Leu
            20                  25                  30

Asp Thr Asn Ile Leu Asn Asn Leu Ile Glu Asn Gln Ile Ser Glu Phe
        35                  40                  45

Glu Leu Pro Met Gly Leu Ala Gln Asn Phe Val Ile Asn Gly Lys Glu
    50                  55                  60

Tyr Ile Val Pro Met Val Thr Glu Glu Pro Ser Val Ile Ala Ala Ala
65                  70                  75                  80

Ser Asn Gly Ala Lys Ile Ala Glu Ser Phe Thr Ala Lys Ile Asp Glu
                85                  90                  95

Arg Leu Met Arg Gly Gln Ile Val Phe Tyr Asp Val Lys Lys Pro Glu
            100                 105                 110

Glu Ile Ile Lys Lys Ile Ser Glu Cys Lys Asn Glu Ile Phe Glu Gln
        115                 120                 125

Ala Lys Leu Ser Tyr Pro Ser Ile Ile Lys Arg Gly Gly Gly Leu Arg
    130                 135                 140

Glu Ile Ser Ser Arg Leu Phe Ser Ser Glu Lys Phe Ile Ser Val Asp
145                 150                 155                 160

Phe Lys Val Asp Val Lys Asp Ala Met Gly Ala Asn Ile Ile Asn Ser
                165                 170                 175

Ile Leu Glu Gly Val Ala Glu Leu Phe Arg Gly Trp Phe Ser Glu Glu
            180                 185                 190

Lys Ile Leu Phe Ser Ile Leu Ser Asn Tyr Ala Thr Glu Ser Leu Val
        195                 200                 205

Lys Val Ser Cys Glu Ile Ser Val Asp Ala Leu Ser Lys Lys Thr Asn
    210                 215                 220

Gly Leu Glu Ile Ala Gln Lys Ile Ala Val Ala Ser Gln Tyr Ser Lys
225                 230                 235                 240

Ile Asp Pro Tyr Arg Ala Ser Thr His Asn Lys Gly Ile Met Asn Gly
                245                 250                 255

Ile Asn Ala Val Ile Leu Ala Thr Gly Asn Asp Thr Arg Ala Ile Ser
            260                 265                 270

Ala Ala Ile His Ala Tyr Ala Ala Lys Glu Gly Thr Tyr Gln Gly Leu
        275                 280                 285

Ala Lys Trp Glu Val His Ala Glu Lys Leu Phe Gly Glu Leu Glu Ile
    290                 295                 300

Pro Leu Pro Val Ala Thr Val Gly Gly Gly Val Lys Val Leu Pro Lys
305                 310                 315                 320

Ala Gln Ala Ala Met Glu Ile Leu Gly Ile Thr Asp Ala Arg Glu Leu
                325                 330                 335

Ala Lys Val Ile Ala Ala Val Gly Leu Ala Gln Asn Leu Ala Ala Leu
            340                 345                 350

Arg Ala Leu Val Ser Glu Gly Ile Gln Gln Gly His Met Ser Leu Gln
        355                 360                 365

Ala Arg Ser Leu Ala Leu Ser Val Gly Ala Lys Ala Asp Glu Ile Ala
    370                 375                 380

Val Ile Ser Gln Gln Leu Arg Gln Glu Lys Val Met Asn Gln Glu Val
385                 390                 395                 400

Ala Arg Arg Leu Leu Asn Ser Leu Arg Asn
                405                 410

<210>  15
<211>  1155
<212>  DNA
<213>  Lactococcus lactis

<220>
<221>  CDS
<222>  (1)..(1155)

<400> 15
atg aaa gtc ggt ata gat aaa ctt gga ttt ttt gtc cca aat act ttt 48
Met Lys Val Gly Ile Asp Lys Leu Gly Phe Phe Val Pro Asn Thr Phe
1               5                   10                  15

gtt gat atg agg gat tta gca aat gct cgt aat gtt gac cct gca aaa 96
Val Asp Met Arg Asp Leu Ala Asn Ala Arg Asn Val Asp Pro Ala Lys
            20                  25                  30

ttc caa att ggt att ggt caa gat gaa atg gca gtc aat cct gct act 144
Phe Gln Ile Gly Ile Gly Gln Asp Glu Met Ala Val Asn Pro Ala Thr
        35                  40                  45

caa gat att att act ttt gct gct aac gcg gca gca agt att ttg act 192
Gln Asp Ile Ile Thr Phe Ala Ala Asn Ala Ala Ala Ser Ile Leu Thr
    50                  55                  60

gag gaa gat aaa aaa gcc att gac atg atc atc gtt ggt act gaa tca 240
Glu Glu Asp Lys Lys Ala Ile Asp Met Ile Ile Val Gly Thr Glu Ser
65                  70                  75                  80

agt ctt gat gaa tca aag gcc tca gcc gtt gtt gtt cac gat ttg ctt 288
Ser Leu Asp Glu Ser Lys Ala Ser Ala Val Val Val His Asp Leu Leu
                85                  90                  95

ggt att caa cct ttt gct cga agc att gaa atg aaa gag gct tgt tat 336
Gly Ile Gln Pro Phe Ala Arg Ser Ile Glu Met Lys Glu Ala Cys Tyr
            100                 105                 110

gca act act gct ggc ctt act ctt gct cgt gac cac gtt ctt ttg aat 384
Ala Thr Thr Ala Gly Leu Thr Leu Ala Arg Asp His Val Leu Leu Asn
        115                 120                 125

cct gat act aaa gtt tta gtt att gct tct gat att gcc aaa tat ggg 432
Pro Asp Thr Lys Val Leu Val Ile Ala Ser Asp Ile Ala Lys Tyr Gly
    130                 135                 140

cta aat act ggt gga gaa cct act cag ggc gct gga agt gtt gca atg 480
Leu Asn Thr Gly Gly Glu Pro Thr Gln Gly Ala Gly Ser Val Ala Met
145                 150                 155                 160

ctt att acg gcc gat cct aaa att ttg gcc tta aat aat gat aac gtt 528
Leu Ile Thr Ala Asp Pro Lys Ile Leu Ala Leu Asn Asn Asp Asn Val
                165                 170                 175

gct tta act caa gac atc tat gat ttt tgg cgt cct ttt ggt cag gct 576
Ala Leu Thr Gln Asp Ile Tyr Asp Phe Trp Arg Pro Phe Gly Gln Ala
            180                 185                 190

tat cct tct gtt gat ggt aag ttt tca aac gaa aca tat atc gat gct 624
Tyr Pro Ser Val Asp Gly Lys Phe Ser Asn Glu Thr Tyr Ile Asp Ala
        195                 200                 205

ttt gct aaa atc tgg aaa gag tat agc cat aga aca aat tta aag ttt 672
Phe Ala Lys Ile Trp Lys Glu Tyr Ser His Arg Thr Asn Leu Lys Phe
    210                 215                 220

gaa gac ttt gca gct atc gcc ttt cat acc cct tat act aaa atg ggt 720
Glu Asp Phe Ala Ala Ile Ala Phe His Thr Pro Tyr Thr Lys Met Gly
225                 230                 235                 240

aaa aaa gct tta ttg cca atg ctt gaa tct gaa aaa cca gcc aat gct 768
Lys Lys Ala Leu Leu Pro Met Leu Glu Ser Glu Lys Pro Ala Asn Ala
                245                 250                 255

gaa gaa tta atg gaa caa ttt gaa cat ggt atc gtt tat aat cgt cgt 816
Glu Glu Leu Met Glu Gln Phe Glu His Gly Ile Val Tyr Asn Arg Arg
            260                 265                 270

gtg ggt aat ctg tat acc ggt tct ctc tat ttg agt ttg att tca ctt 864
Val Gly Asn Leu Tyr Thr Gly Ser Leu Tyr Leu Ser Leu Ile Ser Leu
        275                 280                 285

ttg gaa aac tct gat aaa ttg agt gct ggt cag cgt att gga ctt ttt 912
Leu Glu Asn Ser Asp Lys Leu Ser Ala Gly Gln Arg Ile Gly Leu Phe
    290                 295                 300

agt tat ggt tct ggt act gtt gct gag ttt ttc tct ggt gag ctt gtt 960
Ser Tyr Gly Ser Gly Thr Val Ala Glu Phe Phe Ser Gly Glu Leu Val
305                 310                 315                 320

gaa ggt tat gaa aat cat tta ctt aaa att gaa cat caa tca atg ttg 1008
Glu Gly Tyr Glu Asn His Leu Leu Lys Ile Glu His Gln Ser Met Leu
                325                 330                 335

gat gca aga acc cgt cta tca att cct gaa tac gaa aac atg ttt aat 1056
Asp Ala Arg Thr Arg Leu Ser Ile Pro Glu Tyr Glu Asn Met Phe Asn
            340                 345                 350

caa tat tta gat ttg aat cat aat att tct ttc aat gat gag aca gat 1104
Gln Tyr Leu Asp Leu Asn His Asn Ile Ser Phe Asn Asp Glu Thr Asp

355                 360                 365

tac tcc gtt tca gaa gtt gtt gat aat cac cgt aag tat aaa aaa aga      1152
Tyr Ser Val Ser Glu Val Val Asp Asn His Arg Lys Tyr Lys Lys Arg
    370                 375                 380

taa                                                                  1155

<210>  16
<211>  384
<212>  PRT
<213>  Lactococcus lactis

<400>  16

Met Lys Val Gly Ile Asp Lys Leu Gly Phe Phe Val Pro Asn Thr Phe
1               5                   10                  15

Val Asp Met Arg Asp Leu Ala Asn Ala Arg Asn Val Asp Pro Ala Lys
            20                  25                  30

Phe Gln Ile Gly Ile Gly Gln Asp Glu Met Ala Val Asn Pro Ala Thr
        35                  40                  45

Gln Asp Ile Ile Thr Phe Ala Ala Asn Ala Ala Ala Ser Ile Leu Thr
    50                  55                  60

Glu Glu Asp Lys Lys Ala Ile Asp Met Ile Ile Val Gly Thr Glu Ser
65                  70                  75                  80

Ser Leu Asp Glu Ser Lys Ala Ser Ala Val Val Val His Asp Leu Leu
                85                  90                  95

Gly Ile Gln Pro Phe Ala Arg Ser Ile Glu Met Lys Glu Ala Cys Tyr
            100                 105                 110

Ala Thr Thr Ala Gly Leu Thr Leu Ala Arg Asp His Val Leu Leu Asn
        115                 120                 125

Pro Asp Thr Lys Val Leu Val Ile Ala Ser Asp Ile Ala Lys Tyr Gly
    130                 135                 140

Leu Asn Thr Gly Gly Glu Pro Thr Gln Gly Ala Gly Ser Val Ala Met
145                 150                 155                 160

Leu Ile Thr Ala Asp Pro Lys Ile Leu Ala Leu Asn Asn Asp Asn Val
                165                 170                 175

Ala Leu Thr Gln Asp Ile Tyr Asp Phe Trp Arg Pro Phe Gly Gln Ala
            180                 185                 190

Tyr Pro Ser Val Asp Gly Lys Phe Ser Asn Glu Thr Tyr Ile Asp Ala
        195                 200                 205

Phe Ala Lys Ile Trp Lys Glu Tyr Ser His Arg Thr Asn Leu Lys Phe
    210                 215                 220

Glu Asp Phe Ala Ala Ile Ala Phe His Thr Pro Tyr Thr Lys Met Gly
225                 230                 235                 240

Lys Lys Ala Leu Leu Pro Met Leu Glu Ser Glu Lys Pro Ala Asn Ala
                245                 250                 255

Glu Glu Leu Met Glu Gln Phe Glu His Gly Ile Val Tyr Asn Arg Arg
            260                 265                 270

Val Gly Asn Leu Tyr Thr Gly Ser Leu Tyr Leu Ser Leu Ile Ser Leu
        275                 280                 285

Leu Glu Asn Ser Asp Lys Leu Ser Ala Gly Gln Arg Ile Gly Leu Phe
    290                 295                 300

Ser Tyr Gly Ser Gly Thr Val Ala Glu Phe Phe Ser Gly Glu Leu Val
305                 310                 315                 320

Glu Gly Tyr Glu Asn His Leu Leu Lys Ile Glu His Gln Ser Met Leu
                325                 330                 335

Asp Ala Arg Thr Arg Leu Ser Ile Pro Glu Tyr Glu Asn Met Phe Asn
            340                 345                 350

Gln Tyr Leu Asp Leu Asn His Asn Ile Ser Phe Asn Asp Glu Thr Asp
        355                 360                 365

Tyr Ser Val Ser Glu Val Val Asp Asn His Arg Lys Tyr Lys Lys Arg
    370                 375                 380

<210>  17
<211>  30
<212>  DNA
<213>  Artificial

<220>
<223>  primer E

<400>  17
atgatcgatg gaggttcatt catgaaagaa                                    30

<210>  18
<211>  30
<212>  DNA
<213>  Artificial

<220>
<223>  primer F

<400>  18
agtggatcct tagtttctga gactatttaa                                    30

<210>  19
<211>  26

<212>  DNA
<213>  Artificial

<220>
<223>  primer G

<400>  19
tttagatcta ataagtgagg aaattt                                          26


<210>  20
<211>  30
<212>  DNA
<213>  Artificial

<220>
<223>  primer H

<400>  20
agtggatcct tatctttttt tatacttacg                                      30


<210>  21
<211>  2464
<212>  DNA
<213>  Lactococcus lactis

<220>
<221>  CDS
<222>  (13)..(1152)

<220>
<221>  CDS
<222>  (1232)..(2464)

<400>  21
ggaggttcat tc gtg aaa gaa att gta atc att gac gcc tta aga aca cca      51
              Met Lys Glu Ile Val Ile Ile Asp Ala Leu Arg Thr Pro
              1               5                   10

gtt gga aga tac gac ggt gct ctt gct gaa tat tca gca gca gaa ctt        99
Val Gly Arg Tyr Asp Gly Ala Leu Ala Glu Tyr Ser Ala Ala Glu Leu
    15                  20                  25

ggg aca cat gtt gtt tca aac tta ttg tca aaa cat gaa aat ata aaa       147
Gly Thr His Val Val Ser Asn Leu Leu Ser Lys His Glu Asn Ile Lys

30                  35                  40                  45

tca gat gtt gaa caa gtt att ttt ggg aat gtt ctt caa gct ggt aat      195
Ser Asp Val Glu Gln Val Ile Phe Gly Asn Val Leu Gln Ala Gly Asn
                50                  55                  60

ggt caa aat ata gca cgt caa att gcg att cat agt ggt cta tct aat      243
Gly Gln Asn Ile Ala Arg Gln Ile Ala Ile His Ser Gly Leu Ser Asn
            65                  70                  75

aca gtg ccc gct tca aca atc aac gaa gtt tgt ggt tca gga atg aaa      291
Thr Val Pro Ala Ser Thr Ile Asn Glu Val Cys Gly Ser Gly Met Lys
        80                  85                  90

gca gtg att tta gct aaa caa ctt tta cag ctt ggt gaa gca gaa gtt      339
Ala Val Ile Leu Ala Lys Gln Leu Leu Gln Leu Gly Glu Ala Glu Val
    95                  100                 105

gta atc gct ggt ggg aca gaa tct atg tct aat gcc cca att ata aag      387
Val Ile Ala Gly Gly Thr Glu Ser Met Ser Asn Ala Pro Ile Ile Lys
110                 115                 120                 125

aat cga aaa act caa gaa gaa aca tta agt atg tta aat gat ggc tta      435
Asn Arg Lys Thr Gln Glu Glu Thr Leu Ser Met Leu Asn Asp Gly Leu
                130                 135                 140

gtt gat gcc ttc tca gga att tct atg ggg att att ggt gaa act att      483
Val Asp Ala Phe Ser Gly Ile Ser Met Gly Ile Ile Gly Glu Thr Ile
            145                 150                 155

gct gaa caa ttt gat ata agt cga gca gac caa gac gct ttt gcg caa      531
Ala Glu Gln Phe Asp Ile Ser Arg Ala Asp Gln Asp Ala Phe Ala Gln
        160                 165                 170

aac tct caa gaa aaa gcg gtc gct gca agt caa gcc gga att ttt aaa      579
Asn Ser Gln Glu Lys Ala Val Ala Ala Ser Gln Ala Gly Ile Phe Lys
    175                 180                 185

gaa gag att gtt gcg att gga gaa gtc gga gtc gac gaa aca cca cgt      627
Glu Glu Ile Val Ala Ile Gly Glu Val Gly Val Asp Glu Thr Pro Arg
190                 195                 200                 205

cct tct tct agt tta gaa aaa tta gcc act tta agg aca gct ttc aaa      675
Pro Ser Ser Ser Leu Glu Lys Leu Ala Thr Leu Arg Thr Ala Phe Lys
                210                 215                 220

gaa aat gga act gtg aca gca ggt aat tct tct cct gtt aat gat gga 723
Glu Asn Gly Thr Val Thr Ala Gly Asn Ser Ser Pro Val Asn Asp Gly
            225                 230                 235

gct tca gct gtc att ctt gca acg aaa gac tat gct gaa aaa cat ggg 771
Ala Ser Ala Val Ile Leu Ala Thr Lys Asp Tyr Ala Glu Lys His Gly
        240                 245                 250

att ccg tac cta gca gaa cta gtt gat agt aca gaa gtt gga att gac 819
Ile Pro Tyr Leu Ala Glu Leu Val Asp Ser Thr Glu Val Gly Ile Asp
    255                 260                 265

cct gct att atg gga gtt tca cca att gaa gct atc aaa aaa tta att 867
Pro Ala Ile Met Gly Val Ser Pro Ile Glu Ala Ile Lys Lys Leu Ile
270                 275                 280                 285

aat cgt tca gag ctt gaa ctt tca gat att gac ctg ttt gaa att aat 915
Asn Arg Ser Glu Leu Glu Leu Ser Asp Ile Asp Leu Phe Glu Ile Asn
                290                 295                 300

gaa gca ttt gca gca tca agt ctt gct gtc aat cgt gaa ctt ggt tta 963
Glu Ala Phe Ala Ala Ser Ser Leu Ala Val Asn Arg Glu Leu Gly Leu
            305                 310                 315

aat gaa tca caa gtt aac att tat ggt ggt gca att gca tta ggt cat 1011
Asn Glu Ser Gln Val Asn Ile Tyr Gly Gly Ala Ile Ala Leu Gly His
        320                 325                 330

gct atc ggt tca agt ggt tct aaa att ctc acg act ttg tct tat gcc 1059
Ala Ile Gly Ser Ser Gly Ser Lys Ile Leu Thr Thr Leu Ser Tyr Ala
    335                 340                 345

tta aaa cgt gag caa aaa cgc tat gga att gct agt ctt tgt atc ggt 1107
Leu Lys Arg Glu Gln Lys Arg Tyr Gly Ile Ala Ser Leu Cys Ile Gly
350                 355                 360                 365

gga ggc cta ggt ttg gct gtt ctt tta aaa aat cct aac ctt taa 1152
Gly Gly Leu Gly Leu Ala Val Leu Leu Lys Asn Pro Asn Leu
                370                 375

aaattattgt ggtttaaaca agtaattttt aagaaattaa tggaggaatt gcccagctta 1212

tctgggcatt tttgaaaat atg aga aaa aaa ttt tat caa atg tcg ccc aaa 1264
Met Arg Lys Lys Phe Tyr Gln Met Ser Pro Lys
380                 385                 390

gag cga tta gat tca ttg aat ctg tca gaa gat act cag gaa gtt ttg 1312
Glu Arg Leu Asp Ser Leu Asn Leu Ser Glu Asp Thr Gln Glu Val Leu
                395                 400                 405

agt gaa atg gct tta gat aca aat att tta aac aat cta att gaa aat 1360
Ser Glu Met Ala Leu Asp Thr Asn Ile Leu Asn Asn Leu Ile Glu Asn
            410                 415                 420

caa att tcc gaa ttt gaa ttg cca atg gga ctt gct caa aat ttt gtc 1408
Gln Ile Ser Glu Phe Glu Leu Pro Met Gly Leu Ala Gln Asn Phe Val
        425                 430                 435

att aat gga aaa gaa tac atc gtt ccg atg gtt aca gaa gaa cca tca 1456
Ile Asn Gly Lys Glu Tyr Ile Val Pro Met Val Thr Glu Glu Pro Ser
    440                 445                 450

gtt ata gct gct gcg agt aat gga gca aaa ata gcg gaa agt ttt acg 1504
Val Ile Ala Ala Ala Ser Asn Gly Ala Lys Ile Ala Glu Ser Phe Thr
455                 460                 465                 470

gct aaa ata gat gaa cgg tta atg cgt ggg caa att gta ttt tat gac 1552
Ala Lys Ile Asp Glu Arg Leu Met Arg Gly Gln Ile Val Phe Tyr Asp
                475                 480                 485

gta aag aaa cca gaa gaa att att aag aaa ata tct gaa tgc aaa aat 1600
Val Lys Lys Pro Glu Glu Ile Ile Lys Lys Ile Ser Glu Cys Lys Asn
            490                 495                 500

gaa ata ttt gaa caa gct aag ctt tcg tat cct tca att att aag cgt 1648
Glu Ile Phe Glu Gln Ala Lys Leu Ser Tyr Pro Ser Ile Ile Lys Arg
        505                 510                 515

ggg ggc ggt ttg aga gaa att tcg agc aga ctt ttt tca agt gag aaa 1696
Gly Gly Gly Leu Arg Glu Ile Ser Ser Arg Leu Phe Ser Ser Glu Lys
    520                 525                 530

ttt att tca gtt gat ttc aaa gtg gat gtc aaa gat gca atg ggt gcc 1744
Phe Ile Ser Val Asp Phe Lys Val Asp Val Lys Asp Ala Met Gly Ala
535                 540                 545                 550

aat att att aat tct ata tta gaa ggt gta gca gaa ctt ttt cgt ggc 1792
Asn Ile Ile Asn Ser Ile Leu Glu Gly Val Ala Glu Leu Phe Arg Gly
                555                 560                 565

tgg ttt tct gag gaa aaa att ctg ttt agc atc ctt tca aac tat gca 1840
Trp Phe Ser Glu Glu Lys Ile Leu Phe Ser Ile Leu Ser Asn Tyr Ala

570                 575                 580

act gaa tct tta gta aaa gtg agc tgt gaa att tca gtt gat gct ctt      1888
Thr Glu Ser Leu Val Lys Val Ser Cys Glu Ile Ser Val Asp Ala Leu
        585                 590                 595

tca aaa aaa aca aat ggt tta gaa att gct caa aaa att gca gtt gct      1936
Ser Lys Lys Thr Asn Gly Leu Glu Ile Ala Gln Lys Ile Ala Val Ala
    600                 605                 610

tca caa tac tca aaa att gat cct tac cgt gct agc aca cac aat aaa      1984
Ser Gln Tyr Ser Lys Ile Asp Pro Tyr Arg Ala Ser Thr His Asn Lys
615                 620                 625                 630

ggg att atg aat ggg att aat gct gta att ctt gca aca gga aat gat      2032
Gly Ile Met Asn Gly Ile Asn Ala Val Ile Leu Ala Thr Gly Asn Asp
                635                 640                 645

acg cga gct att tca gca gca att cat gct tat gca gca aaa gaa gga      2080
Thr Arg Ala Ile Ser Ala Ala Ile His Ala Tyr Ala Ala Lys Glu Gly
            650                 655                 660

aca tat caa ggt ttg gct aaa tgg gaa gtt cat gcc gaa aag tta ttt      2128
Thr Tyr Gln Gly Leu Ala Lys Trp Glu Val His Ala Glu Lys Leu Phe
        665                 670                 675

gga gag tta gaa att cct tta cct gtg gcg acc gtt ggt ggt ggg gtg      2176
Gly Glu Leu Glu Ile Pro Leu Pro Val Ala Thr Val Gly Gly Gly Val
    680                 685                 690

aaa gtc tta cct aaa gca caa gcg gca atg gaa att tta gga atc aca      2224
Lys Val Leu Pro Lys Ala Gln Ala Ala Met Glu Ile Leu Gly Ile Thr
695                 700                 705                 710

gat gct aga gaa ctg gcc aaa gtt atc gcc gca gtt ggt tta gca caa      2272
Asp Ala Arg Glu Leu Ala Lys Val Ile Ala Ala Val Gly Leu Ala Gln
                715                 720                 725

aat tta gca gct ttg cgc gca ctt gtt tca gaa gga att caa caa gga      2320
Asn Leu Ala Ala Leu Arg Ala Leu Val Ser Glu Gly Ile Gln Gln Gly
            730                 735                 740

cat atg agc tta cag gct cgc tct ttg gca tta tcc gtg ggt gca aaa      2368
His Met Ser Leu Gln Ala Arg Ser Leu Ala Leu Ser Val Gly Ala Lys
        745                 750                 755

gca gat gag ata gca gtc ata agt cag caa ctt cgg cag gaa aaa gtg     2416
Ala Asp Glu Ile Ala Val Ile Ser Gln Gln Leu Arg Gln Glu Lys Val
    760                 765                 770

atg aat caa gaa gta gct cgc cgt tta tta aat agt ctc aga aac taa     2464
Met Asn Gln Glu Val Ala Arg Arg Leu Leu Asn Ser Leu Arg Asn
775                 780                 785

<210>  22
<211>  1179
<212>  DNA
<213>  Lactococcus lactis

<220>
<221>  CDS
<222>  (25)..(1179)

<400>  22
aataagtgag gaaattttta aaat atg aaa gtc ggt ata gat aaa ctt gga       51
                           Met Lys Val Gly Ile Asp Lys Leu Gly
                           1               5

ttt ttt gtc cca aat act ttt gtt gat atg agg gat tta gca aat gct       99
Phe Phe Val Pro Asn Thr Phe Val Asp Met Arg Asp Leu Ala Asn Ala
10                  15                  20                  25

cgt aat gtt gac cct gca aaa ttc caa att ggt att ggt caa gat gaa      147
Arg Asn Val Asp Pro Ala Lys Phe Gln Ile Gly Ile Gly Gln Asp Glu
                30                  35                  40

atg gca gtc aat cct gct act caa gat att att act ttt gct gct aac      195
Met Ala Val Asn Pro Ala Thr Gln Asp Ile Ile Thr Phe Ala Ala Asn
            45                  50                  55

gcg gca gca agt att ttg act gag gaa gat aaa aaa gcc att gac atg      243
Ala Ala Ala Ser Ile Leu Thr Glu Glu Asp Lys Lys Ala Ile Asp Met
        60                  65                  70

atc atc gtt ggt act gaa tca agt ctt gat gaa tca aag gcc tca gcc      291
Ile Ile Val Gly Thr Glu Ser Ser Leu Asp Glu Ser Lys Ala Ser Ala
    75                  80                  85

gtt gtt gtt cac gat ttg ctt ggt att caa cct ttt gct cga agc att      339
Val Val Val His Asp Leu Leu Gly Ile Gln Pro Phe Ala Arg Ser Ile
90                  95                  100                 105

gaa atg aaa gag gct tgt tat gca act act gct ggc ctt act ctt gct 387
Glu Met Lys Glu Ala Cys Tyr Ala Thr Thr Ala Gly Leu Thr Leu Ala
                110                 115                 120

cgt gac cac gtt ctt ttg aat cct gat act aaa gtt tta gtt att gct 435
Arg Asp His Val Leu Leu Asn Pro Asp Thr Lys Val Leu Val Ile Ala
            125                 130                 135

tct gat att gcc aaa tat ggg cta aat act ggt gga gaa cct act cag 483
Ser Asp Ile Ala Lys Tyr Gly Leu Asn Thr Gly Gly Glu Pro Thr Gln
        140                 145                 150

ggc gct gga agt gtt gca atg ctt att acg gcc gat cct aaa att ttg 531
Gly Ala Gly Ser Val Ala Met Leu Ile Thr Ala Asp Pro Lys Ile Leu
    155                 160                 165

gcc tta aat aat gat aac gtt gct tta act caa gac atc tat gat ttt 579
Ala Leu Asn Asn Asp Asn Val Ala Leu Thr Gln Asp Ile Tyr Asp Phe
170                 175                 180                 185

tgg cgt cct ttt ggt cag gct tat cct tct gtt gat ggt aag ttt tca 627
Trp Arg Pro Phe Gly Gln Ala Tyr Pro Ser Val Asp Gly Lys Phe Ser
                190                 195                 200

aac gaa aca tat atc gat gct ttt gct aaa atc tgg aaa gag tat agc 675
Asn Glu Thr Tyr Ile Asp Ala Phe Ala Lys Ile Trp Lys Glu Tyr Ser
            205                 210                 215

cat aga aca aat tta aag ttt gaa gac ttt gca gct atc gcc ttt cat 723
His Arg Thr Asn Leu Lys Phe Glu Asp Phe Ala Ala Ile Ala Phe His
        220                 225                 230

acc cct tat act aaa atg ggt aaa aaa gct tta ttg cca atg ctt gaa 771
Thr Pro Tyr Thr Lys Met Gly Lys Lys Ala Leu Leu Pro Met Leu Glu
    235                 240                 245

tct gaa aaa cca gcc aat gct gaa gaa tta atg gaa caa ttt gaa cat 819
Ser Glu Lys Pro Ala Asn Ala Glu Glu Leu Met Glu Gln Phe Glu His
250                 255                 260                 265

ggt atc gtt tat aat cgt cgt gtg ggt aat ctg tat acc ggt tct ctc 867
Gly Ile Val Tyr Asn Arg Arg Val Gly Asn Leu Tyr Thr Gly Ser Leu
                270                 275                 280

tat ttg agt ttg att tca ctt ttg gaa aac tct gat aaa ttg agt gct 915

Tyr Leu Ser Leu Ile Ser Leu Leu Glu Asn Ser Asp Lys Leu Ser Ala
            285                 290                 295

ggt cag cgt att gga ctt ttt agt tat ggt tct ggt act gtt gct gag 963
Gly Gln Arg Ile Gly Leu Phe Ser Tyr Gly Ser Gly Thr Val Ala Glu
        300                 305                 310

ttt ttc tct ggt gag ctt gtt gaa ggt tat gaa aat cat tta ctt aaa 1011
Phe Phe Ser Gly Glu Leu Val Glu Gly Tyr Glu Asn His Leu Leu Lys
    315                 320                 325

att gaa cat caa tca atg ttg gat gca aga acc cgt cta tca att cct 1059
Ile Glu His Gln Ser Met Leu Asp Ala Arg Thr Arg Leu Ser Ile Pro
330                 335                 340                 345

gaa tac gaa aac atg ttt aat caa tat tta gat ttg aat cat aat att 1107
Glu Tyr Glu Asn Met Phe Asn Gln Tyr Leu Asp Leu Asn His Asn Ile
                350                 355                 360

tct ttc aat gat gag aca gat tac tcc gtt tca gaa gtt gtt gat aat 1155
Ser Phe Asn Asp Glu Thr Asp Tyr Ser Val Ser Glu Val Val Asp Asn
            365                 370                 375

cac cgt aag tat aaa aaa aga taa 1179
His Arg Lys Tyr Lys Lys Arg
        380